(19) Europäisches Patentamt
European Patent Office
Office européen des brevets

(11) EP 2 349 186 B1

(12) # FASCICULE DE BREVET EUROPEEN

(45) Date de publication et mention de la délivrance du brevet:
**21.05.2014 Bulletin 2014/21**

(21) Numéro de dépôt: **09783774.4**

(22) Date de dépôt: **06.10.2009**

(51) Int Cl.:
***A61K 8/04*** (2006.01) ***C08J 3/11*** (2006.01)
***C08L 33/00*** (2006.01) ***A61K 8/90*** (2006.01)
***C08F 12/14*** (2006.01) ***C08F 287/00*** (2006.01)
***C08F 293/00*** (2006.01) ***C08F 2/14*** (2006.01)
***A61K 8/81*** (2006.01) ***A61Q 1/00*** (2006.01)
***A61Q 3/00*** (2006.01) ***A61Q 5/00*** (2006.01)
***A61Q 17/04*** (2006.01) ***A61Q 19/00*** (2006.01)

(86) Numéro de dépôt international:
**PCT/EP2009/062947**

(87) Numéro de publication internationale:
**WO 2010/046229 (29.04.2010 Gazette 2010/17)**

(54) **DISPERSION DE PARTICULES SOUPLES DE POLYMÈRE, COMPOSITION COSMÉTIQUE LA COMPRENANT ET PROCÉDÉ DE TRAITEMENT COSMÉTIQUE**

DISPERSION VON WEICHEN POLYMERTEILCHEN, DIESE ENTHALTENDE KOSMETISCHE ZUSAMMENSETZUNG UND KOSMETISCHES BEHANDLUNGSVERFAHREN

DISPERSION OF SOFT POLYMER PARTICLES, COSMETIC COMPOSITION COMPRISING IT AND COSMETIC TREATMENT METHOD

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **24.10.2008 FR 0857236**
**30.10.2008 US 109514 P**

(43) Date de publication de la demande:
**03.08.2011 Bulletin 2011/31**

(73) Titulaire: **L'Oréal**
**75008 Paris (FR)**

(72) Inventeurs:
- **FARCET, Céline**
**F-93320 Les Pavillons sous Bois (FR)**
- **HOUILLOT, Lisa**
**68165 Mannheim (DE)**
- **SAVE, Maud**
**F-65250 Escala (FR)**
- **CHARLEUX, Bernadette**
**F-69003 Lyon (FR)**

(74) Mandataire: **Kromer, Christophe et al**
**L'Oréal**
**D.I.P.I.**
**25-29 Quai Aulagnier**
**92665 Asnières-sur-Seine (FR)**

(56) Documents cités:
**FR-A1- 2 785 530 GB-A- 941 305**
**US-A- 4 098 980**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 349 186 B1

Printed by Jouve, 75001 PARIS (FR)

## Description

La présente invention a trait à de nouvelles compositions cosmétiques comprenant des dispersions de particules de polymères, auxdites dispersions et à leur utilisation en cosmétique, notamment pour obtenir un dépôt brillant et agréable à porter.

Il est connu d'utiliser en cosmétique des dispersions de particules de polymère, généralement de taille nanométrique, dans des milieux aqueux ou organiques. Ainsi, dans la demande de brevet européen EP-A-0749747, il est décrit une composition cosmétique comprenant une dispersion de particules de polymère, dans un milieu organique, ladite dispersion étant stabilisée par ajout de polymères stabilisants qui se lient de manière non covalente par le biais d'interactions physiques sur les particules de polymère. Ce type de composition présente toutefois comme inconvénient de nécessiter l'ajout dans le milieu organique d'une quantité de polymère stabilisant supérieure à celle effectivement liée aux particules de polymères, afin d'obtenir une dispersion relativement stable. Or, lors de l'ajout d'adjuvants tels que des pigments, une partie des polymères stabilisants a tendance à se désorber des particules de polymères pour s'associer avec lesdits adjuvants, ce qui contribue à déstabiliser la dispersion, notamment par formation d'agglomérats entre les particules de polymères.

On a alors proposé, pour s'exempter de l'utilisation de tels polymères stabilisants, des compositions cosmétiques comprenant des dispersions, dans un milieu organique, de particules de polymères acryliques comprenant un squelette insoluble dans ledit milieu, et une partie soluble dans ledit milieu constituée de chaînes latérales liées de manière covalente audit squelette. Ceci est notamment décrit dans la demande EP1428844. Dans ce cas, les particules de polymères sont stabilisées par un macromonomère qui est chimiquement lié aux particules de polymères. Toutefois, les dispersions décrites dans ces documents, si elles conduisent à des dépôts filmogènes cosmétiquement acceptables, ne permettent pas d'obtenir des propriétés cosmétiques optimales, notamment en terme de glissant, de non collant, de confort, de tenue de la composition, de toucher et de brillance.

En particulier, les films obtenus à partir de ces dispersions présentent un certain caractère collant ainsi qu'une sensibilité aux corps gras, notamment au cours du temps.

Or, on recherche, notamment dans le domaine du maquillage, des polymères susceptibles de présenter une résistance et une tenue aux agressions externes, notamment aux 'agressions' par des corps gras, tels que par exemple par l'huile alimentaire ou le sébum, tout en étant souple et brillant.

La présente invention a pour but de pallier ces limitations et de proposer des dispersions, en milieu huileux, de particules de polymères permettant d'obtenir de bonnes propriétés cosmétiques telles qu'une bonne adhésion sur le support (peau ou cheveu), donc une bonne tenue de la composition cosmétique, une bonne résistance aux agressions, une bonne brillance, tout en étant non collant.

Un objet de la présente invention est une dispersion de particules de polymère, dans un milieu carboné liquide, lesdites particules présentant un coeur polymérique souple, et étant stabilisées en surface par un polymère stabilisant séquencé comprenant au moins une séquence soluble dans ledit milieu carboné et au moins une séquence insoluble dans ledit milieu carboné.

Un autre objet de l'invention est une composition cosmétique comprenant, dans un milieu cosmétiquement acceptable, au moins une dispersion telle que définie ci-dessus.

Les dispersions de particules de polymères selon l'invention possèdent des propriétés cosmétiques adéquates, et notamment une bonne tenue de la composition cosmétique, une bonne stabilité dans le temps. Le confort de la composition cosmétique est amélioré, ainsi que son toucher, sa douceur et son glissant, et son caractère non collant. La composition permet d'obtenir un film brillant et peu collant, tout en étant en outre peu ou non cassant et suffisamment souple, et présentant avantageusement une bonne résistance aux corps gras.

Les compositions selon l'invention peuvent apporter, dans le domaine du maquillage, des propriétés de confort accrues, notamment un glissant amélioré, en particulier en environnement humide. De plus, elles peuvent présenter une résistance améliorée aux agressions externes (huile, repas, sébum) et aux frottements. Le confort et la tenue sont donc améliorés. Les dispersions présentent également une grande affinité pour les milieux huileux usuellement employés en cosmétique. En outre, elles peuvent présenter un taux élevé de matière sèche tout en conservant leur stabilité.

Par ailleurs, ces dispersions comprennent un dispersant en très faible quantité et de nature proche de celle du polymère formant la particule; les propriétés finales du film, par exemple la Tg, ne seront donc pas, ou peu, affectées par le dispersant contrairement à ce qui peut se passer avec l'utilisation de dispersants classiques qui peuvent venir perturber les propriétés finales, notamment de part leur nature chimique et/ou la quantité employée plus importante (en général au moins 10%).

Pour atteindre le but poursuivi par la présente invention, il a été nécessaire de concevoir à la fois le coeur de la particule et son stabilisant, de manière spécifiquement adaptée.

Ainsi, selon la présente invention, on souhaite préparer des particules dites souples qui vont former un dépôt sur le substrat kératinique; lors du séchage de la dispersion, après évaporation du milieu organique, lesdites particules peuvent coalescer et former un film.

De plus, le milieu de la dispersion doit être un milieu carboné liquide.

On a constaté que le choix particulier des monomères susceptibles de former les particules selon l'invention, ainsi que le choix particulier du milieu carboné liquide, pouvait permettre en outre d'obtenir une dispersion stable et susceptible de comprendre les particules en grande quantité, conduisant à une dispersion présentant un taux de matière sèche important.

Afin d'obtenir une telle dispersion, il est proposé de polymériser des monomères particuliers, susceptibles de former le coeur polymérique dit 'souple' de la particule, en présence d'un polymère stabilisant séquencé comprenant au moins une séquence soluble et au moins une séquence insoluble dans le milieu de dispersion, ledit stabilisant formant en partie les 'cheveux' de la particule.

Ainsi que mentionné ci-dessus, pour obtenir un dépôt filmogène, souple et confortable à porter, ainsi que brillant, les particules en dispersion, et plus particulièrement le coeur desdites particules, est/sont de préférence souple.

De préférence, lesdites particules ne sont pas, ou peu, réticulées, de manière à conserver leur caractère filmogène.

Lesdites particules présentant un coeur polymérique souple peuvent être obtenues par polymérisation de monomères, seuls ou en mélange, choisis de telle manière que la température de transition vitreuse (Tg) du polymère en résultant, formant le coeur souple, est strictement inférieur à 20°C.

Dans la présente invention, les Tg (ou température de transition vitreuse) indiquées sont des Tg théoriques déterminées à partir des Tg théoriques des monomères constitutifs du polymère, que l'on peut trouver dans un manuel de référence, tel que le Polymer Handbook, 4th éd. (Brandrup, Immergut, Grulke), 1999, John Wiley. La Tg d'un polymère peut être déterminée selon la relation suivante, dite Loi de Fox :

$$\frac{1}{Tg} = \sum_i \left(\frac{\varpi i}{Tgi}\right)$$

wi étant la fraction massique du monomère i dans le polymère et Tgi étant la température de transition vitreuse de l'homopolymère du monomère i (exprimée en Kelvin).

Dans la présente description, on désigne par "monomère de Tg", le monomère dont l'homopolymère a une telle température de transition vitreuse.

Afin d'obtenir des particules à coeur polymérique souple, dont la Tg est strictement inférieure à 20°C, il est possible de polymériser 60 à 99,9% en poids, par rapport au poids total de monomères, de monomères dont l'homopolymère a une Tg strictement inférieure à 20°C, notamment 80-95% en poids de monomères de Tg strictement inférieure à 20°C; préférentiellement, 100% en poids des monomères susceptibles de former le coeur de la particule sont de Tg strictement inférieure à 20°C. Les monomères dont l'homopolymère a une Tg supérieure ou égale à 20°C (monomères de Tg supérieure ou égale à 20°C) peuvent donc être présents pour former le coeur de la particule, mais en une quantité minoritaire, c'est-à-dire comprise entre 0,1 et 40% en poids, notamment 5 à 20% en poids, du poids total de monomères servant à former le coeur de la particule.

Le polymère susceptible de former le coeur polymérique de la particule peut être obtenu par polymérisation radicalaire conventionnelle ou polymérisation radicalaire contrôlée (PRC), notamment en milieu carboné liquide; cette dernière technique est parfaitement adaptée car elle permet le contrôle de la taille des particules dans ces milieux et de leur dispersité.

De préférence, les monomères susceptibles de former le coeur polymérique de la particule sont choisis parmi les monomères insolubles dans le milieu carboné liquide de la dispersion.

Par monomère insoluble, on entend dans la présente description tout monomère dont l'homopolymère est insoluble, à 5% en poids, à 20°C, dans le milieu carboné liquide de la dispersion; le monomère en tant que tel pouvant lui être soluble ou insoluble dans ledit milieu.

Les monomères insolubles représentent notamment 55 à 100% en poids, en particulier 65 à 95% en poids, voire 70 à 85% en poids, du poids total de monomères formant le coeur polymérique de la particule.

Comme monomère de Tg strictement inférieure à 20°C, susceptible d'être employé pour former le coeur polymérique de la particule, on peut citer, seul ou en mélange, les monomères suivants, ainsi que leurs sels :

- les (méth)acrylates de formule $CH_2=C(CH_3)-COOR$ ou $CH_2=CH-COOR$, dans laquelle R représente un groupe alkyle, linéaire, ramifié ou cyclique, saturé ou insaturé, voire aromatique, comprenant 1 à 32 atomes de carbone, pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4; et/ou interrompu par un atome d'oxygène ;
- les monomères éthyléniques dont le groupement ester contient des silanes, des silsesquioxanes, des siloxanes, des carbosiloxanes dendrimères tels que décrits dans le brevet EP 0 963 751, à l'exception des monomères ne

contenant qu'un atome de silicium tel que le méthacryloxypropryl trimethoxysilane.

Parmi les monomères de Tg strictement inférieure à 20°C plus particulièrement préférés, on peut citer :

- l'acrylate de perfluorooctyle, de butyle, d'isobutyle, de méthyle, de méthoxyéthyle, de cyclohexyle, d'éthoxy-2-éthyle;
- le méthacrylate de diméthylaminoéthyle, d'éthoxy-2-éthyle;
- le (méth)acryloxypropyltris(trimethylsiloxy)silane, le 3-(méth)acryloxypropyl-bis(trimethylsiloxy)méthylsilane, le (méth)acryloxyméthyltris(trimethylsiloxy)silane, le (méth)acryloxyméthylbis(trimethylsiloxy)méthylsilane.

Comme monomère de Tg supérieure ou égale à 20°C, susceptible d'être employé pour former partie du coeur de la particule, on peut citer, seul ou en mélange, les monomères suivants, ainsi que leurs sels :

- (i) les (méth)acrylates de formule $CH_2=C(CH_3)-COOR$ ou $CH_2=CH-COOR$, dans laquelle R représente un groupe alkyle, linéaire, ramifié ou cyclique, saturé ou insaturé, voire aromatique, comprenant 1 à 32 atomes de carbone, pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4;
  et notamment :
  - le méthacrylate de méthyle, d'éthyle, de cyclohexyle, d'isobutyle, de butyle, de tertiobutyle, de tétrahydrofurfuryle, de dicyclopentenyloxyéthyle, de benzyle;
  - l'acrylate de tertiobutyle,
  - le méthacrylate de trifluoroéthanol (MATRIFE)
  - les (méth)acrylates de 2-hydroxyéthyle, de 2-hydroxypropyle, de dicyclopentenyle,
- (ii) les (méth)acrylamides de formule $CH_2=C(CH_3)-CONR'_3R'_4$ ou $CH_2=CH-CONR'_3R'_4$ dans laquelle :
  - R'3 et R'4, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, =O, les atomes d'halogène (F, Cl, Br, I) et -NR'R" avec R' et R", identiques ou différents, choisis parmi les alkyles, linéaires ou ramifiés, en C1-C4; ou
  - R'3 représente un atome d'hydrogène et R'4 représente un groupe 1,1-diméthyl-3-oxobutyle ;
    A titre d'exemples de groupes alkyles pouvant constituer R'3 et R'4, on peut citer le n-butyle, le t-butyle, le n-propyle, le diméthylaminoéthyle, le diéthylaminoéthyle, le diméthylaminopropyle.
    Comme monomère, on peut citer le diméthylaminopropylméthacrylamide; l'acrylamide, le méthacrylamide, la N-tertbutylacrylamide, la diacetoneacrylamide de formule $CH_2=CH-C(O)NHC(CH_3)_2-CH_2-C(O)CH_3$;
- (iii) les monomères à insaturation(s) éthylénique(s) comprenant au moins une fonction acide carboxylique, phosphorique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylique, l'acide méthacrylique, l'acide acrylamidopropanesulfonique, l'acide acrylamidoglycolique, et leurs sels;
- (iv) les esters de vinyle de formule R'6-COO-CH=CH2 dans laquelle R'6 représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique;
- (v) les monomères à insaturation(s) éthylénique(s) comprenant au moins une fonction amine tertiaire, tel que la 2-vinylpyridine, la 4-vinylpyridine, et leurs mélanges;
- (vi) le styrène et ses dérivés;

Parmi les monomères de Tg supérieure ou égale à 20°C plus particulièrement préférés, on peut citer l'acide méthacrylique, l'acide acrylique; le méthacrylate de méthyle, d'éthyle, de cyclohexyle, d'isobutyle, de butyle, de tertiobutyle, de tétrahydrofurfuryle, de dicyclopentenyloxyéthyle, de benzyle; l'acrylate de tertiobutyle; le méthacrylate de trifluoroéthanol; les (méth)acrylates de 2-hydroxyéthyle, de 2-hydroxypropyle, de dicyclopentenyle; le diméthylaminopropylméthacrylamide; le styrène et ses dérivés, l'acétate de vinyle; et leurs mélanges.

Une caractéristique de la présente invention est que la polymérisation des monomères formant le coeur de la particule est effectuée en présence d'un polymère stabilisant séquencé, ce qui va conduire à la stabilisation en surface desdites particules par ledit polymère stabilisant.

Ledit polymère stabilisant séquencé est de préférence un copolymère tel que décrit dans la demande EP1704854. Il comprend au moins une première séquence (ou bloc) soluble dans le milieu carboné liquide de la dispersion et au moins une deuxième séquence (ou bloc) insoluble dans ledit milieu.

Par séquence, on entend dans la présente invention, un enchaînement polymérique, formé de plusieurs monomères, notamment d'au moins 5 monomères, identiques ou différents, qui peut donc se présenter sous forme d'un homopolymère ou d'un copolymère statistique, alterné, à gradient ou bloc, notamment dibloc, tribloc ou multibloc. De préférence, la séquence est de type homopolymère ou à gradient, préférentiellement homopolymère. Pour chaque séquence, le choix des monomères et de leur quantité, ainsi que de l'architecture de la séquence, peut être effectué par l'homme du métier sur la base de ses connaissances générales de manière à obtenir au final une séquence ayant la solubilité requise (soluble ou insoluble) dans le milieu carboné considéré. Chaque séquence peut être de longueur, de masse molaire, de nature chimique et/ou d'architecture différente ou identique. Par soluble, on entend que la séquence est complètement dissoute (sans dépôt apparent, ni agglomérat ou sédiment insoluble), visuellement, à 20°C, à une concentration supérieure ou égale à 5% en poids, dans le milieu carboné considéré.

Le polymère stabilisant séquencé est de préférence du type 'tribloc', c'est-à-dire qu'il comprend trois séquences, et est notamment du type tribloc soluble/insoluble/soluble; il peut toutefois être du type 'dibloc' du type soluble/insoluble, voire 'multiblocs' (plus de trois blocs).

De préférence, le polymère stabilisant séquencé est linéaire; il peut toutefois être ramifié, greffé et/ou branché. On entend par linéaire un polymère pour lequel il n'y a pas, lors de sa polymérisation, d'ajout volontaire de composé ayant pour but de le réticuler et/ou de le ramifier.

De préférence, le polymère stabilisant séquencé a un poids moléculaire moyen en nombre (Mn) compris entre 1000 à 700 000, notamment entre 5000 et 500 000, et encore mieux entre 10 000 et 350 000, voire entre 15 000 et 150 000. De préférence, il présente un indice de polydispersité en masse (Ip) inférieur ou égal à 6, de préférence compris entre 1,05 et 4, notamment entre 1,1 et 3, voire entre 1,15 et 2,5. L'indice de polydispersité en masse (Ip) est égal au rapport de la masse moléculaire moyenne en poids (Mw) sur la masse moléculaire moyenne en nombre (Mn). On détermine les masses moléculaires moyennes en poids (Mw) et en nombre (Mn) par chromatographie liquide par perméation de gel (GPC), éluant THF, courbe d'étalonnage établie avec des étalons de polystyrène linéaire, détecteur réfractométrique ou détecteur diffusion de la lumière.

Le polymère stabilisant séquencé comprend une première séquence soluble dans le milieu carboné de dispersion et au moins une seconde séquence, insoluble dans ledit milieu.

La séquence soluble comprend de préférence 50 à 100% en poids de monomère soluble dans ledit milieu, notamment de 60 à 90% en poids, et encore mieux de 70 à 80% en poids de monomère soluble, seul ou en mélange. Elle peut donc comprendre également 0 à 50% en poids, notamment de 10 à 40% en poids, voire de 20 à 30% en poids de monomère insoluble dans ledit milieu, seul ou en mélange. De manière similaire, la séquence insoluble comprend de préférence 50 à 100% en poids de monomère insoluble dans ledit milieu, notamment de 60 à 90% en poids, et encore mieux de 70 à 80% en poids de monomère insoluble, seul ou en mélange. Elle peut comprendre également 0 à 50% en poids, notamment de 10 à 40% en poids, voire de 20 à 30% en poids de monomère soluble dans ledit milieu, seul ou en mélange.

Par monomère soluble dans le milieu, on entend tout monomère dont l'homopolymère est sous forme soluble c'est-à-dire complètement dissous à une concentration supérieure ou égale à 5% en poids à température ambiante (20°C) dans ledit milieu.

Par monomère insoluble, on entend donc tout monomère dont l'homopolymère n'est pas sous forme soluble c'est-à-dire complètement dissous à une concentration supérieure ou égale à 5% en poids à température ambiante (20°C) dans ledit milieu. Toutefois, les monomères dits insolubles peuvent, en tant que monomères, être solubles dans le milieu considéré, étant entendu qu'ils deviennent insolubles après polymérisation.

Comme monomère soluble susceptible d'être employé pour former tout ou partie de la séquence soluble, on peut citer, seul ou en mélange, les monomères suivants :

- les méthacrylates de formule $CH_2 = C(CH_3)\text{-}COOR_1$
  dans laquelle $R_1$ représente un groupe alkyle linéaire ou ramifié en C8-C22 tel que lauryle, béhényle ou stéaryle; ou bien un groupe alkyle cyclique ayant 8 à 30 atomes de carbone, tel que isobornyle; ou encore $R_1$ représente le groupe tertiobutyle;
- les acrylates de formule $CH_2 = CH\text{-}COOR_2$
  dans laquelle $R_2$ représente un groupe alkyle linéaire ou ramifié en C8-C22 tel que lauryle, béhényle ou stéaryle ou 2-éthylhexyle; ou bien un groupe alkyle cyclique ayant 8 à 30 atomes de carbone, tel que isobornyle; ou encore $R_2$ représente un groupe isobutyle ;
- les (méth)acrylamides de formule $CH_2{=}C(CH_3)\text{-}CONR_3R_4$ ou $CH_2{=}CH\text{-}CONR_3R_4$, dans lesquelles $R_3$ représente un atome d'hydrogène ou un groupe alkyle linéaire ou ramifié en C1-C12, et $R_4$ représente un groupe alkyle en $C_8$ à $C_{12}$ linéaire ou ramifié, tel qu'un groupe isooctyle, isononyle, undecyle;

- les di-n-alkylitaconates de formule $CH_2=C(CH_2-COO(CH_2)_{n-1}-CH_3)-COO(CH_2)_{n-1}-CH_3$, avec n étant un entier supérieur ou égal à 5, notamment de 5 à 12;
- les esters de vinyle de formule $R_5$-CO-O-CH=$CH_2$ où $R_5$ représente un groupe alkyle en $C_8$ à $C_{22}$ linéaire ou ramifié ;
- les éthers d'alcool vinylique et d'alcool de formule $R_6$O-CH=$CH_2$ dans laquelle $R_6$ représente un groupe alkyle linéaire ou ramifié, comprenant de 8 à 22 atomes de carbone;
- les monomères éthyléniques dont le groupement ester contient des silanes ou des siloxanes, et ne contenant qu'un atome de silicium tel que le (méth)acryloxypropyl trimethoxysilane;
- des macromonomères carbonés ayant un groupe terminal polymérisable.

On entend par "macromonomère ayant un groupe terminal polymérisable" tout oligomère comportant sur une seule de ses extrémités un groupe terminal polymérisable apte à réagir lors de la réaction de polymérisation avec des monomères éthyléniques. Le groupe polymérisable du macromonomère peut être avantageusement un groupe à insaturation éthylénique susceptible de se polymériser par voie radicalaire. Ledit groupe terminal polymérisable peut être en particulier un groupe vinyle ou (méth)acrylate (ou (méth)acryloxy), et de préférence un groupe (méth)acrylate. Par "macromonomère carboné" on entend un macromonomère non siliconé, et notamment un macromonomère oligomère obtenu par polymérisation de monomère(s) non siliconé(s) à insaturation éthylénique, et principalement par polymérisation de monomères acryliques et/ou vinyliques non acryliques. Comme macromonomères carbonés ayant un groupe terminal polymérisable, on peut en particulier citer :

- (i) les homopolymères et les copolymères (méth)acrylate d'alkyle linéaire ou ramifié en C6-C22, de préférence en C8-C18, présentant un groupe terminal polymérisable choisi parmi les groupes vinyle ou (méth)acrylate parmi lesquels on peut citer en particulier: les macromonomères de poly(acrylate d'éthyl-2 hexyle) à extrémité mono(méth)acrylate; les macromonomères de poly(acrylate de dodécyle) ou de poly(méthacrylate de dodécyle) à extrémité mono(méth)acrylate ; les macromonomères de poly(acrylate de stéaryle) ou de poly (méthacrylate de stéaryle) à extrémité mono(méth)acrylate.
  De tels macromonomères sont notamment décrits dans les brevets EP895467 et EP96459 et dans l'article Gillman , Polymer Letters, Vol 5, page 477-481 (1967). On peut en particulier citer les macromonomères à base de poly(acrylate d'éthyl-2-hexyle) ou de poly(acrylate de dodécyle) à extrémité mono(méth)acrylate.
- (ii) les polyoléfines ayant un groupe terminal à insaturation éthylénique, en particulier ceux ayant un groupement terminal (méth)acrylate. Comme exemple de telles polyoléfines, on peut citer en particulier les macromonomères suivants, étant entendu qu'ils ont un groupe terminal (méth)acrylate : les macromonomères de polyéthylène, les macromonomères de polypropylène, les macromonomères de copolymère polyéthylène/polypropylène, les macromonomères de copolymère polyéthylène/polybutylène, les macromonomères de polyisobutylène ; les macromonomères de polybutadiène; les macromonomères de polyisoprène ; les macromonomères de polybutadiène; les macromonomères de poly(éthylène/butylène)-polyisoprène ;
  De tels macromonomères sont en particulier décrits dans EP1347013 ou encore dans US 5,625,005 qui mentionne des macromonomères éthylène/butylène et éthylène/propylène à groupement terminal réactif (méth)acrylate. On peut en particulier citer le méthacrylate de poly(éthylène/butylène), tel que celui commercialisé sous la dénomination Kraton Liquid L-1253 par Kraton Polymers.

Comme monomère soluble particulièrement préféré, on peut citer, seul ou en mélange:

- les méthacrylates de formule $CH_2 = C(CH_3)-COOR_1$
  dans laquelle $R_1$ représente un groupe alkyle linéaire ou ramifié en C8-C22 tel que lauryle, béhényle ou stéaryle; ou bien un groupe alkyle cyclique ayant 8 à 30 atomes de carbone, tel que isobornyle; ou encore $R_1$ représente le groupe tertiobutyle;
- les acrylates de formule $CH_2 = CH-COOR_2$
  dans laquelle $R_2$ représente un groupe alkyle linéaire ou ramifié en C8-C22 tel que lauryle, béhényle, 2-éthylhexyle ou stéaryle; ou bien un groupe alkyle cyclique ayant 8 à 30 atomes de carbone, tel que isobornyle; ou encore $R_2$ représente un groupe isobutyle.
  On peut ainsi citer l'acrylate d'éthyle-2-hexyle, le (méth)acrylate d'isobornyle, le (méth)acrylate de lauryle, le (méth)acrylate de stéaryle, le (méth)acrylate de béhényle, l'acrylate d'isobutyle et le méthacrylate de tertiobutyle, et leurs mélanges.

Comme monomère insoluble susceptible d'être employé notamment pour former tout ou partie de la séquence insoluble, on peut citer, seul ou en mélange, les monomères suivants, ainsi que leurs sels et leurs mélanges:

- (i) les (méth)acrylates de formule : $CH_2=C(CH_3)-COOR'_1$ ou $CH_2=CH-COOR'_1$ dans laquelle R'1 représente un

groupe choisi parmi :

- un groupe alkyle, linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4; et/ou pouvant être substitué par au moins un groupe polyoxyalkylène, en particulier avec un alkylène en C2-C4, notamment un polyoxyéthylène et/ou un polyoxy-propylène, ledit groupe polyoxyalkylène étant constitué par la répétition de 5 à 30 motifs oxyalkylène; sont exclus de cette définition le méthacrylate de tertiobutyle et l'acrylate d'isobutyle.
- un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène (F, Cl, Br, I);

A titre d'exemples de R'1, on peut citer le groupe méthyle, éthyle, propyle, butyle, méthoxyéthyle, éthoxyéthyle, méthoxy-polyoxyéthylène 30, trifluoroéthyle, 2-hydroxyéthyle, 2-hydroxypropyle, diméthylaminoéthyle, diéthylaminoéthyle, diméthylaminopropyle.

- (ii) les (méth)acrylamides de formule : $CH_2=C(CH_3)-CONR'_3R'_4$ ou $CH_2=CH-CONR'_3R'_4$, dans laquelle :

  - R'3 et R'4, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène (F, Cl, Br, I) et -NR'R" avec R' et R", identiques ou différents, choisis parmi les alkyles, linéaires ou ramifiés, en C1-C4;ou
  - R'3 représente un atome d'hydrogène et R'4 représente un groupe 1,1-diméthyl-3-oxobutyle ;

  A titre d'exemples de groupes alkyles pouvant constituer R'3 et R'4, on peut citer le n-butyle, le t-butyle, le n-propyle, le diméthylaminoéthyle, le diéthylaminoéthyle, le diméthylaminopropyle.

- (iii) les monomères à insaturation(s) éthylénique(s) comprenant au moins une fonction acide carboxylique, phosphorique ou sulfonique, tel que l'acide crotonique, l'anhydride maléique, l'acide itaconique, l'acide fumarique, l'acide maléique, l'acide styrènesulfonique, l'acide vinylbenzoïque, l'acide vinylphosphorique, l'acide acrylique, l'acide méthacrylique, l'acide acrylamidopropanesulfonique, et leurs sels;

- (iv) les esters de vinyle de formule R'6-COO-CH=CH2 dans laquelle R'6 représente un groupe alkyle linéaire ou ramifié, comprenant de 1 à 6 atomes, ou un groupe alkyle cyclique comportant de 3 à 6 atomes de carbone et/ou un groupe aromatique, par exemple de type benzénique, anthracénique, et naphtalénique ;

- (v) les monomères à insaturation(s) éthylénique(s) comprenant au moins une fonction amine tertiaire, tel que la 2-vinylpyridine, la 4-vinylpyridine, et leurs mélanges.

- (vi) les di-n-alkylitaconates de formule $CH_2=C(CH_2-COO(CH_2)_{n-1}-CH_3)-COO(CH_2)_{n-1}-CH_3$, avec n étant un entier de 0 à 4;

- (vii) les monomères éthyléniques dont le groupement ester contient des silanes, des silsesquioxanes, des siloxanes, des carbosiloxanes dendrimères tels que décrits dans le brevet EP 0 963 751, à l'exception des monomères ne contenant qu'un atome de silicium tel que le méthacryloxypropryl trimethoxysilane. Des monomères préférés sont le (méth)acryloxypropyltris(trimethylsiloxy)silane, le (méth)acryloxypropylbis(trimethylsiloxy)méthylsilane, le (méth)acryloxyméthyltris(tri-méthylsiloxy) silane, et le (méth)acryloxyméthylbis(trimethylsiloxy)méthylsilane.

- (viii) les macromonomères PDMS tels que les polydiméthylsiloxanes à groupement terminal monoacryloyloxy ou monométhacryloyloxy, et notamment eux de formule suivante :

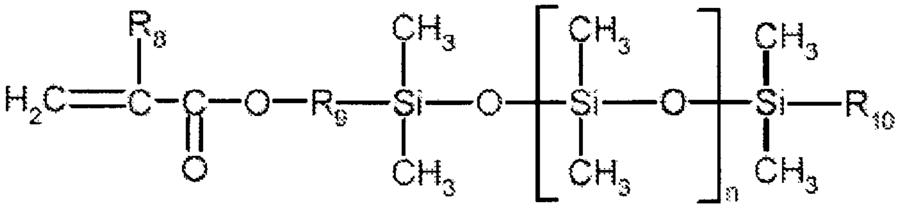

dans laquelle :

- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;
- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

On peut en particulier utiliser les monométhacryloyloxypropyl polydiméthylsiloxanes tels que ceux commercialisés sous la dénomination PS560-K6 par UCT (United Chemical Technologies Inc.) ou sous la dénomination MCR-M17 par Gelest Inc.

Parmi les sels, on peut citer ceux obtenus par neutralisation des groupements acides à l'aide de base inorganiques telles que l'hydroxyde de sodium, l'hydroxyde de potassium, l'hydroxyde d'ammonium ou de bases organiques de type alcanols amines comme la monoéthanolamine, la diéthanolamine, la triéthanolamine, la 2-méthyl-2-amino-1-propanol. On peut également citer les sels formés par neutralisation des motifs amine tertiaire, par exemple à l'aide d'acide minéral ou organique. Parmi les acides minéraux, on peut citer l'acide sulfurique ou l'acide chlorhydrique, l'acide bromhydrique, iodhydrique, l'acide phosphorique, l'acide borique. Parmi les acides organiques, on peut citer les acides comportant un ou plusieurs groupes carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles. On peut notamment citer l'acide acétique ou l'acide propionique, l'acide téréphtalique, ainsi que l'acide citrique et l'acide tartrique.

Comme monomère insoluble particulièrement préféré, on peut citer :

- les (méth)acrylates de formule : $CH_2=C(CH_3)-COOR'_1$, ou $CH_2=CH-COOR'_1$, et en particulier les (méth)acrylates de méthyle, d'éthyle, de propyle, de butyle; le méthacrylate d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle;
- les monomères à insaturation(s) éthylénique(s) comprenant au moins une fonction acide carboxylique, et notamment l'acide (méth)acrylique et ses sels;
- les monomères éthyléniques dont le groupement ester contient des silanes;
- les polydiméthylsiloxanes à groupement terminal monoacryloyloxy ou monométhacryloyloxy, de formule suivante :

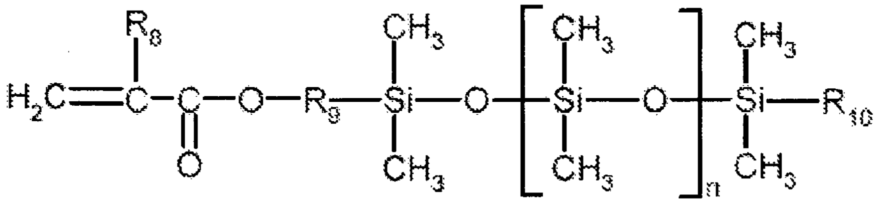

dans laquelle :

- R8 désigne un atome d'hydrogène ou un groupement méthyle; de préférence méthyle;
- R9 désigne un groupe hydrocarboné divalent, linéaire ou ramifié, de préférence linéaire, ayant de 1 à 10 atomes de carbone et contenant éventuellement une ou deux liaisons éther -O- ; de préférence éthylène, propylène ou butylène;

- R10 désigne un groupe alkyle linéaire ou ramifié, ayant de 1 à 10 atomes de carbone, notamment de 2 à 8 atomes de carbone; de préférence méthyle, éthyle, propyle, butyle ou pentyle;
- n désigne un nombre entier allant de 1 à 300, de préférence allant de 3 à 200, et préférentiellement allant de 5 à 100.

Plus particulièrement, on peut citer le (méth)acrylate de méthyle, le (méth)acrylate d'éthyle, l'acide (méth)acrylique, l'anhydride maléïque, le (méth)acryloxypropyltris(trimethylsiloxy)silane, le (méth)acryloxypropyl bis(trimethylsiloxy)méthylsilane, le (méth)acryloxyméthyltris(trimethylsiloxy)silane et le (méth)acryloxyméthylbis-(trimethylsiloxy)méthylsilane.

On préfèrera tout particulièrement les polymères stabilisants séquencés dans lesquels :

(i) la séquence soluble comprend 50-100% en poids de monomères choisis parmi, seul ou en mélange:

- les méthacrylates de formule $CH_2 = C(CH_3)\text{-}COOR_1$ dans laquelle $R_1$ représente un groupe alkyle linéaire ou ramifié en C8-C22 tel que lauryle, béhényle ou stéaryle; ou bien un groupe alkyle cyclique ayant 8 à 30 atomes de carbone, tel que isobornyle; ou bien $R_1$ représente le groupe tertiobutyle;
- les acrylates de formule $CH_2 = CH\text{-}COOR_2$ dans laquelle $R_2$ représente un groupe alkyle linéaire ou ramifié en C8-C22 tel que lauryle, béhényle, 2-éthylhexyle ou stéaryle; ou bien un groupe alkyle cyclique ayant 8 à 30 atomes de carbone, tel que isobornyle; ou bien $R_2$ représente un groupe isobutyle; et

(ii) la séquence insoluble comprend 50-100% en poids de monomères choisis parmi , seuls ou en mélange, les (méth)acrylates de méthyle, d'éthyle, de propyle, de butyle; le méthacrylate d'isobutyle; les (méth)acrylates de méthoxyéthyle ou d'éthoxyéthyle; le méthacrylate de trifluoroéthyle; le méthacrylate de diméthylaminoéthyle, le méthacrylate de diéthylaminoéthyle, le méthacrylate de 2-hydroxypropyle, le méthacrylate de 2-hydroxyéthyle, l'acrylate de 2-hydroxypropyle, l'acrylate de 2-hydroxyéthyle; l'acide (méth)acrylique et ses sels;

Parmi les polymères stabilisants préférés, on peut tout particulièrement citer les polymères suivants: le poly(acrylate de 2-éthyle hexyle)-b-poly(acrylate de méthyle), le poly(acrylate de 2-éthyle hexyle)-b-poly(acrylate de méthyle-co-acide acrylique), le poly(acrylate de 2-éthyle hexyle-co-acrylate d'isobornyle)-b-poly(acrylate de méthyle), le poly(acrylate de 2-éthyle hexyle-co-acide acrylique)-b-poly(acrylate de méthyle), le poly(acrylate de 2-éthyle hexyle-co-acrylate d'isobornyle)-b-poly(acrylate de méthyle-co-acide acrylique), le poly(acrylate de 2-éthyle hexyle-co-acrylate d'isobornyle-co-acide acrylique)-b-poly(acrylate de méthyle); le poly(acrylate d'isobornyle)-b-poly(acrylate de méthyle), le poly(acrylate d'isobornyle)-b-poly(acrylate de méthyle-co-acide acrylique); le poly(acrylate d'isobornyle-co-acide acrylique)-b-poly(acrylate de méthyle), le poly(acrylate d'isobutyle)-b-poly(acrylate de méthyle), le poly(acrylate d'isobutyle)-b-poly(acrylate de méthyle-co-acide acrylique), le poly(acrylate d'isobutyle-co-acide acrylique)-b-poly(acrylate de méthyle), le poly(acrylate d'isobutyle-co-acrylate d'isobornyle)-b-poly(acrylate de méthyle), le poly(acrylate d'isobutyle-co-acrylate d'isobornyle)-b-poly(acrylate de méthyle-co-acide acrylique), le poly(acrylate d'isobutyle-co-acrylate d'isobornyle-co-acide acrylique)-b-poly(acrylate de méthyle), le poly(acrylate de 2-éthyle hexyle)-b-poly(acrylate de méthyle)-b-poly(acrylate de 2-éthyle hexyle), le poly(acrylate de 2-éthyle hexyle-co-acrylate d'isobornyle)-b-poly(acrylate de méthyle)-b-poly(acrylate de 2-éthyle hexyle-co-acrylate d'isobornyle), le poly(acrylate de 2-éthyle hexyle-co-acide acrylique)-b-poly(acrylate de méthyle)-b- poly(acrylate de 2-éthyle hexyle-co-acide acrylique), le poly(acrylate de 2-éthyle hexyle)-b-poly(acrylate de méthyle-co-acide acrylique)-b-poly(acrylate de 2-éthyle hexyle), le poly(acrylate de 2-éthyle hexyle-co-acrylate d'isobornyle-co-acide acrylique)-b-poly(acrylate de méthyle)-b-poly(acrylate de 2-éthyle hexyle-co-acrylate d'isobornyle-co-acide acrylique), le poly(acrylate de 2-éthyle hexyle-co-acrylate d'isobornyle)-b-poly(acrylate de méthyle-co-acide acrylique)-b-poly(acrylate de 2-éthyle hexyle-co-acrylate d'isobornyle).

Le polymère stabilisant selon l'invention peut être préparé par l'homme du métier selon toutes les techniques de polymérisation connue; de préférence, la première séquence est préparée par polymérisation radicalaire contrôlée (PRC), la seconde séquence pouvant être également préparée par PRC ou par polymérisation conventionnelle.

La dispersion de particules de polymères selon l'invention comprend également un milieu carboné liquide dans lequel sont dispersées lesdites particules.

Par milieu liquide, on entend notamment un milieu ayant de préférence une viscosité inférieure ou égale à 7000 centipoises à 20°C.

Selon l'invention, le milieu est dit carboné, s'il comprend au moins 50% en poids, notamment de 50 à 100% en poids, par exemple de 60 à 99% en poids, ou encore de 65 à 95% en poids, voire de 70 à 90% en poids, par rapport au poids total du milieu carboné, de composé carboné, liquide à 25°C, ayant un paramètre de solubilité global selon l'espace de solubilité de HANSEN inférieur ou égal à 20 $(MPa)^{1/2}$, ou d'un mélange de tels composés.

Le paramètre de solubilité global $\delta$ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = ( d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).

La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés carbonés liquides ayant un paramètre de solubilité global selon l'espace de solubilité de HANSEN inférieur ou égal à 20(MPa)$^{1/2}$, on peut citer les corps gras liquides, notamment les huiles, qui peuvent être choisies parmi les huiles naturelles ou synthétiques, carbonées, hydrocarbonées, éventuellement fluorées, éventuellement ramifiées, seules ou en mélange.

En particulier, on peut citer :

- les huiles végétales formées par des esters d'acides gras et de polyols, en particulier les triglycérides, telles que l'huile de tournesol, de sésame ou de colza, de macadamia, de soja, l'huile d'amande douce, de calophyllum, de palme, de pépins de raisin, de maïs, d'arara, de coton, d'abricot, d'avocat, de jojoba, d'olive ou de germes de céréales;
- les esters linéaires, ramifiés ou cycliques, ayant plus de 6 atomes de carbone, notamment 6 à 30 atomes de carbone; et notamment l'isononanoate d'isononyle ; et plus particulièrement les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle ;
- les hydrocarbures et notamment les alcanes linéaires, ramifiés et/ou cycliques, volatils ou non volatils, tels que les isoparaffines en $C_5$-$C_{60}$, éventuellement volatiles tels que l'isododécane, le Parléam (polyisobutène hydrogéné), l'isohexadécane, le cyclohexane, ou les 'ISOPARs'; ou bien les huiles de paraffine, de vaseline, ou le polyisobutylène hydrogéné.
- les éthers ayant 6 à 30 atomes de carbone ;
- les cétones ayant 6 à 30 atomes de carbone.
- les monoalcools gras aliphatiques ayant 6 à 30 atomes de carbone, la chaîne hydrocarbonée ne comportant pas de groupement de substitution, tels que l'alcool oléique, le décanol, le dodécanol, l'octadécanol, l'octyldodécanol et l'alcool linoléique;
- les polyols ayant 6 à 30 atomes de carbone, tels que l'hexylène glycol;
- leurs mélanges.

De préférence, la dispersion comprend dans le milieu carboné, au moins un composé carboné choisi parmi :

- les huiles végétales formées par des esters d'acides gras et de polyols, en particulier les triglycérides,
- les esters de formule RCOOR' dans laquelle R représente le reste d'un acide gras supérieur comportant 7 à 19 atomes de carbone et R' représente une chaîne hydrocarbonée comportant de 3 à 20 atomes de carbone,
- les alcanes linéaires ou ramifiés en C8-C60, volatils ou non volatils;
- les alcanes cycliques, non aromatiques, en C5-C12; volatils ou non volatils;
- les éthers ayant 7 à 30 atomes de carbone;
- les cétones ayant 8 à 30 atomes de carbone;
- les monoalcools gras aliphatiques ayant 12 à 30 atomes de carbone, la chaîne hydrocarbonée ne comportant pas de groupement de substitution,
- leurs mélanges.

Préférentiellement, le milieu carboné comprend au moins, comme composé carboné, du myristate d'isopropyle, de l'octyldodécanol, des isoparaffines en C5-C60, de l'isododécane, de l'isohexadécane, de l'isononanoate d'isononyle,

du Parléam.

Le milieu carboné peut éventuellement comprendre des composés liquides additionnels qui peuvent être présents en une quantité strictement inférieure à 50% en poids, notamment de 1 à 40% en poids, voire de 5 à 35% en poids, ou encore de 10 à 30% en poids, par rapport au poids total du milieu carboné, et choisis parmi, seul ou en mélange :

- les huiles siliconées, volatiles ou non volatiles, seules ou en mélange ;
  On peut notamment citer les polydiméthylsiloxanes et les polyméthylphénylsiloxanes, éventuellement substitués par des groupements aliphatiques et/ou aromatiques, éventuellement fluorés, et/ou comportant des groupements fonctionnels tels que des groupements hydroxyles, thiols et/ou amines; et les siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodécaméthylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.
- les esters ayant 2 à 5 atomes de carbone, les éthers ayant 2 à 6 atomes de carbone, les cétones 1 à 5 atomes de carbone, les monoalcools ayant 1 à 5 atomes de carbone.
  Toutefois, selon un mode particulier de réalisation de l'invention, le milieu carboné ne contient pas de composés liquides additionnels.

Le choix du milieu carboné peut être effectué aisément par l'homme du métier en fonction de la nature des monomères constituant le polymère et/ou de la destination de la composition.

La dispersion de particules de polymères, stabilisée par un polymère stabilisant séquencé, peut être préparée selon toutes les méthodes envisageables par l'homme du métier, sur la base de ses connaissances générales.

Dans un mode de préparation particulier, on prépare, dans une première étape, le polymère stabilisant séquencé, sous forme de dispersion ou sous forme sèche, puis on synthétise, dans une seconde étape, les particules de polymère en présence dudit polymère stabilisant. Lors de sa synthèse, ledit polymère stabilisant séquencé, en dispersion dans le milieu, peut s'organiser spontanément et former des micelles polymériques autodispersées, en dispersion stable dans le milieu. Ces micelles (ou particules micellaires) sont de préférence d'une taille comprise entre 5 et 500 nm, notamment entre 10 et 400 nm, encore mieux de 20 à 200 nm, voire de 30 à 100 nm. De préférence, elles sont toutes de la même taille. Préférentiellement, on prépare une dispersion du polymère stabilisant séquencé, dans le milieu carboné souhaité pour la dispersion finale, et l'on effectue la polymérisation des monomères susceptibles de former le coeur de la particule, en présence de cette dispersion. La taille finale des particules est de préférence supérieure à 100 nm, et elles sont de préférence toutes de même taille.

Le polymère stabilisant séquencé, notamment en dispersion, peut être préparé par polymérisation radicalaire contrôlée ou par polymérisation vivante, notamment par les techniques dites nitroxydes/alcoxyamines, ATRP, aux organocobalt, RAFT/MADIX, transfert dégénératif, TERP (tellurium), au Selenium, par Iniferter, ou par tout procédé de polymérisation vivante (anionique ou cationique), par métallocène, par ROMP (ring opening metathesis polymerization), par ROP (ring opening polymerization) cationique ou anionique, par GTP (group transfer polymerization), par les dérivés du tétraphenyléthane, par le diphénylethylène. Les techniques employées pour la formation de chaque séquence peuvent être identiques ou différentes.

Un procédé type peut consister à préparer la première séquence, dite soluble, dans le milieu carboné de la dispersion, par polymérisation du ou des monomères, d'un agent de contrôle et d'un amorceur si nécessaire. Ensuite, le ou les monomères de la séquence dite insoluble sont ajoutés en présence ou non d'amorceur. La température de réaction est de préférence comprise entre -30 et 200°C, de préférence de 0 à 160°C et plus préférentiellement de 40 à 140°C. Des séquences supplémentaires peuvent être polymérisées selon le même procédé. Pour chacune des séquences, le ou les monomères peuvent être ajoutés simultanément, en batch, en semi-continu ou consécutivement. On obtiendra alors des polymères multiblocs. Si la première séquence, dite soluble, est synthétisée en masse, la séquence dite insoluble peut être ensuite synthétisée en masse ou en solution. Le solvant peut être un solvant carboné, ce qui conduit à l'issue de la synthèse du copolymère à une dispersion dans le milieu carboné. Le solvant employé peut également être un solvant commun à toutes les séquences; dans ce cas, l'ajout ultérieur d'un solvant carboné et l'élimination éventuelle du solvant commun conduira à l'éventuelle dispersion. Si tout le copolymère est synthétisé en masse, l'ajout d'un solvant carboné conduira à l'éventuelle dispersion. Si toutes les séquences sont synthétisées en solution, dans un solvant commun, l'ajout ultérieur d'un solvant carboné et l'élimination éventuelle du solvant commun conduira à l'éventuelle dispersion. Il est également possible à ce stade d'éliminer le solvant commun afin de récupérer le polymère seul pour l'utiliser tel quel ou le disperser dans un solvant carboné afin d'obtenir une dispersion. Enfin, si toutes les séquences sont synthétisées directement dans un solvant carboné, la dispersion est obtenue directement, en une seule étape. De préférence, la première séquence du polymère stabilisant est préparée par polymérisation radicalaire contrôlée (PRC), la seconde séquence pouvant être également préparée par PRC ou par polymérisation conventionnelle.

L'amorceur de polymérisation peut être tout amorceur connu de l'homme de l'art pour la polymérisation radicalaire

(peroxydes, azoïques, couple rédox, photochimique). Dans le cas de certaines techniques de polymérisation radicalaire contrôlée, un même composé peut avoir pour rôle d'amorcer la polymérisation et d'être l'agent de contrôle comme c'est le cas des alcoxyamines. Pour les polymérisations non radicalaires, c'est-à-dire ioniques (anionique ou cationique), l'homme de l'art peut choisir l'amorceur adéquat.

Quel que soit le mode de préparation du polymère stabilisant, on obtient au final des polymères stabilisants séquencés qui s'auto-organisent lorsqu'ils sont en dispersion dans le milieu considéré. Ils sont composés d'au moins une séquence soluble et d'au moins une séquence insoluble, ce qui va provoquer une auto-organisation des chaînes de polymères de manière à former des micelles présentant à l'interface avec le milieu les séquences solubles et au coeur de la micelle les séquences insolubles.

Dans une deuxième étape, on peut polymériser les monomères susceptibles de former le coeur de la particule, en présence dudit polymère stabilisant. Sans être tenu par la présente explication, on peut considérer que les micelles de polymère stabilisant vont s'adsorber sur les particules de polymère en formation; lesdits monomères vont en particulier polymériser au coeur desdites micelles et les faire grossir, et conduire au final à des particules souples très monodisperses, de diamètre préférentiellement supérieur à 100 nm. Cette seconde étape peut être une polymérisation conventionnelle ou une polymérisation radicalaire contrôlée.

Dans un autre mode de préparation de la dispersion selon l'invention, mode tout particulièrement préféré, on peut synthétiser en une seule étape les particules de polymère en dispersion, avec synthèse in situ du polymère stabilisant séquencé. On forme ainsi en même temps le polymère stabilisant et le coeur des particules. Dans ce cas également, on peut considérer que les monomères du coeur de la particule vont de préférence polymériser au coeur des micelles en formation, afin de conduire au final à des particules souples très monodisperses, de diamètre préférentiellement supérieur à 100 nm. Dans ce mode de préparation, la polymérisation est de préférence radicalaire contrôlée.
Dans ce mode de préparation, au moins l'un des blocs du polymère stabilisant séquencé, de préférence le bloc insoluble, est de nature chimique identique au coeur de la particule, c'est-à-dire comprenant les mêmes monomères; on peut penser que ceci est dû à la formation concomitante du bloc insoluble du polymère stabilisant séquencé et du coeur de la particule.

On peut schématiser comme suit les deux modes décrits ci-dessus, de formation des particules en dispersion selon l'invention :

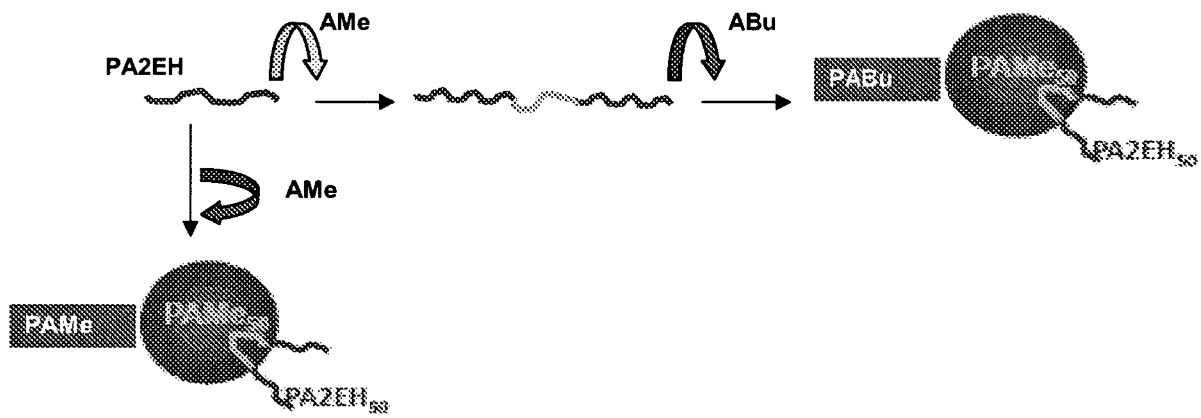


Dans le 1er mode (flèche horizontale) selon cet exemple, on forme tout d'abord un premier bloc soluble de poly(acrylate d'éthyle 2-hexyle) (PA2EH), puis un polymère tribloc de poly(acrylate d'éthyle 2-hexyle)-b-polyacrylate de méthyle-b-poly(acrylate d'éthyle 2-hexyle); on polymérise ensuite de l'acrylate de butyle (ABu) en présence du tribloc qui s'est préalablement organisé en micelles, et l'on obtient au final, en dispersion, une particule souple de poly(acrylate de butyle) stabilisée par ledit tribloc.
Dans le 2ème mode (flèche verticale) selon cet exemple, on forme tout d'abord un premier bloc soluble de poly(acrylate d'éthyle 2-hexyle) (PA2EH), puis de manière concomitante, le polymère tribloc de poly(acrylate d'éthyle 2-hexyle)-b-poly(acrylate de méthyle)-b-poly(acrylate d'éthyle 2-hexyle) qui s'organise en micelles et le polyacrylate de méthyle (AMe) qui forme le coeur de la particule; on obtient au final, en dispersion, une particule souple de poly(acrylate de méthyle) stabilisée par ledit tribloc.

Le rapport en poids entre d'une part les monomères servant à former le/les blocs solubles du polymère stabilisant séquencé, et d'autre part les monomères formant le coeur de la particule + les monomères formant le/les blocs insolubles du polymère stabilisant, est de préférence compris entre 0,5/100 et 15/100 (soit 0,5 à 15 parties en poids de monomères servant à former les blocs solubles pour 100 parties en poids des monomères servant à former les blocs insolubles + le coeur de la particule), notamment entre 1 /100 et 10/100, voire entre 1,5/100 et 8/100.

La dispersion de particules de polymères selon l'invention est très préférentiellement monodisperse, ce qui signifie qu'elle présente une polydispersité en taille des particules très faible, notamment inférieure ou égale à 0,15, de préférence inférieure ou égale à 0,12; la polydispersité en taille des particules (PDI) peut notamment être déterminée par diffusion dynamique de la lumière, à l'aide d'un Malvern Zetasizer Nano S90; on emploie des cuves en polystyrène (1 cm x 1 cm) et les mesures sont effectuées à 20°C, sur des solutions comprenant 1 goutte de dispersion diluée dans 1 ml d'isododécane.

Les particules de polymère en dispersion selon l'invention ont de préférence une forme sphérique et un diamètre supérieur à 100 nm, notamment compris entre 100 et 1000 nm, en particulier entre 125 et 800 nm, préférentiellement entre 150 et 700 nm (déterminé par diffusion dynamique de la lumière, à l'aide d'un Malvern Zetasizer Nano S90).

De préférence, la dispersion selon l'invention présente un taux de matière sèche de 5 à 40% en poids, notamment de 6 à 30% en poids, voire de 7 à 25% en poids.

Les dispersions selon l'invention trouvent une application toute particulière en cosmétique. Ainsi, elles peuvent être présentes dans les compositions cosmétiques selon l'invention en une quantité telle que la quantité de matière sèche (ou matière active) de polymère dans la composition représente 1 à 70% en poids, de préférence 2 à 50% en poids, notamment 5 à 40% en poids, voire 7 à 30% en poids, par rapport au poids total de la composition.

Les compositions cosmétiques selon l'invention comprennent en outre, un milieu cosmétiquement acceptable, c'est-à-dire compatible avec les matières kératiniques telles que la peau du visage ou du corps, les lèvres, les cheveux, les cils, les sourcils et les ongles.

La composition peut avantageusement comprendre une phase grasse, qui peut elle-même comprendre des huiles et/ou des solvants de préférence lipophiles, ainsi que des corps gras solides à température ambiante tels que les cires, les corps gras pâteux, les gommes et leurs mélanges.

Parmi les constituants de la phase grasse, on peut citer les huiles, volatiles ou non, qui peuvent être choisies parmi les huiles naturelles ou synthétiques, carbonées, hydrocarbonées, fluorées, éventuellement ramifiées, seules ou en mélange. On entend par "huile non volatile", une huile susceptible de rester sur la peau à température ambiante et pression atmosphérique au moins une heure et ayant notamment une pression de vapeur à température ambiante (25°C) et pression atmosphérique, non nulle, inférieure à 0,01 mm de Hg (1,33 Pa).

On peut en particulier citer les huiles non volatile carbonées, notamment hydrocarbonées, d'origine végétale, minérale, animale ou synthétique, telles que l'huile de paraffine (ou vaseline), le squalane, le polyisobutène hydrogéné (Parléam), le perhydrosqualène, l'huile de vison, de macadamia, de tortue, de soja, l'huile d'amande douce, de calophyllum, de palme, de pépins de raisin, de sésame, de maïs, d'arara, de colza, de tournesol, de coton, d'abricot, de ricin, d'avocat, de jojoba, d'olive ou de germes de céréales, de beurre de karité; les esters linéaires, ramifiés ou cycliques, ayant plus de 6 atomes de carbone, notamment 6 à 30 atomes de carbone, tels que les esters d'acide lanolique, d'acide oléique, d'acide laurique, d'acide stéarique; les esters dérivés d'acides ou d'alcools à longue chaîne (c'est-à-dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, en particulier les esters en C12-C36, tels que le myristate d'isopropyle, le palmitate d'isopropyle, le stéarate de butyle, le laurate d'hexyle, l'adipate de diisopropyle, l'isononanoate d'isononyle, le palmitate de 2-éthyl-hexyle, le laurate de 2-hexyl-décyle, le palmitate de 2-octyl-décyle, le myristate ou le lactate de 2-octyl-dodécyle, le succinate de di(2-éthyl hexyle), le malate de diisostéaryle, le triisostéarate de glycérine ou de diglycérine; les acides gras supérieurs, notamment en C14-C22, tels que l'acide myristique, l'acide palmitique, l'acide stéarique, l'acide béhénique, l'acide oléique, l'acide linoléique, l'acide linolénique ou l'acide isostéarique; les alcools gras supérieurs, notamment en C16-C22, tels que le cétanol, l'alcool oléique, l'alcool linoléique ou linolénique, l'alcool isostéarique ou l'octyl dodécanol; et leurs mélanges.

On peut encore citer le décanol, le dodécanol, l'octadécanol, les triglycérides liquides d'acides gras de 4 à 10 atomes de carbone comme les triglycérides des acides heptanoïque ou octanoïque, les triglycérides des acides caprylique/caprique; les hydrocarbures linéaires ou ramifiés, d'origine minérale ou synthétique tels que les huiles de paraffine et leurs dérivés, la vaseline, les polydécènes, le polyisobutène hydrogéné tel que le parléam; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras; des esters de polyol comme le dioctanoate de propylène glycol, le diheptanoate de néopentylglycol, le diisononanoate de diéthylèneglycol ; et les esters du pentaérythritol; des alcools gras ayant de 12 à 26 atomes de carbone comme l'octyldodécanol, le 2-butyloctanol, le 2-hexyldécanol, le 2-undécylpentadécanol.

On peut encore citer les cétones liquides à température ambiante tels que méthyléthylcétone, méthylisobutylcétone, diisobutylcétone, l'isophorone, la cyclohexanone, l'acétone; les éthers de propylène glycol liquides à température ambiante tels que le monométhyléther de propylène glycol, l'acétate de monométhyléther de propylène glycol, le mono n-butyl éther de dipropylène glycol ; les esters à chaîne courte (ayant de 3 à 8 atomes de carbone au total) tels que

l'acétate d'éthyle, l'acétate de méthyle, l'acétate de propyle, l'acétate de n-butyle, l'acétate d'isopentyle; les éthers liquides à température ambiante tels que le diéthyléther, le diméthyléther ou le dichlorodiéthyléther; les alcanes liquides à température ambiante tels que le décane, l'heptane, le dodécane, l'isododécane, l'isohexadécane, le cyclohexane; les composés cycliques aromatiques liquides à température ambiante tels que le toluène et le xylène; les aldéhydes liquides à température ambiante tels que le benzaldéhyde, l'acétaldéhyde et leurs mélanges.
Parmi les composés volatils, on peut citer les huiles volatiles non siliconées, notamment les isoparaffines en C8-C16 comme l'isododécane, l'isodécane, l'isohexadécane. Plus préférentiellement, on peut citer les alcanes liquides à température ambiante, volatils ou non, et plus particulièrement le décane, l'heptane, le dodécane, l'isododécane, l'isohexadécane, le cyclohexane, l'isodécane, et leurs mélanges.
La phase grasse peut être présente en une teneur allant de 0,01 à 95%, de préférence de 0,1 à 90%, de préférence encore de 10 à 85% en poids, par rapport au poids total de la composition, et mieux de 30 à 80%.

La composition peut également comprendre, une phase hydrophile comprenant de l'eau ou un mélange d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le pentylène glycol, et les polyéthylène glycols, ou bien encore des éthers en $C_2$ et des aldéhydes en $C_2$-$C_4$ hydrophiles. L'eau ou le mélange d'eau et de solvants organiques hydrophiles peut être présent dans la composition selon l'invention en une teneur allant de 0,1 à 80% en poids, par rapport au poids total de la composition, et de préférence de 1 à 70% en poids.

La composition selon l'invention peut également comprendre des cires et/ou des gommes. Par cire au sens de la présente invention, on entend un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles éventuellement présentes et de former un mélange homogène microscopiquement, mais en ramenant la température du mélange à la température ambiante, on obtient une recristallisation de la cire dans les huiles du mélange. Le point de fusion de la cire peut être mesuré à l'aide d'un calorimètre à balayage différentiel (D.S.C.), par exemple le calorimètre vendu sous la dénomination DSC 30 par la société METLER.
Les cires peuvent être hydrocarbonées, fluorées et/ou siliconées et être d'origine végétale, minérale, animale et/ou synthétique. En particulier, les cires présentent une température de fusion supérieure à 25°C et mieux supérieure à 45°C. Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.
Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.
La nature et la quantité des corps solides sont fonction des propriétés mécaniques et des textures recherchées. A titre indicatif, la composition peut contenir de 0,01 à 50% en poids de cires, par rapport au poids total de la composition et mieux de 1 à 30 % en poids.

La composition selon l'invention peut en outre comprendre une ou des matières colorantes choisies parmi les colorants hydrosolubles, les colorants liposolubles, et les matières colorantes pulvérulentes comme les pigments, les nacres, et les paillettes bien connues de l'homme du métier. Les matières colorantes peuvent être présentes, dans la composition, en une teneur allant de 0,01 à 50% en poids, par rapport au poids de la composition, de préférence de 0,01 à 30% en poids. Par pigments, il faut comprendre des particules de toute forme, blanches ou colorées, minérales ou organiques, insolubles dans le milieu physiologique, destinées à colorer la composition. Par nacres, il faut comprendre des particules de toute forme irisées, notamment produites par certains mollusques dans leur coquille ou bien synthétisées. Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre. Parmi les pigments organiques, on peut citer le noir de carbone, les pigments de type D & C, et les laques à base de carmin de cochenille, de baryum, strontium, calcium, aluminium. Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.
Parmi les colorants hydrosolubles, on peut citer le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle, le bleu de méthylène.

La composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition. Les charges peuvent être minérales ou organiques de toute forme, plaquettaires, sphériques ou oblongues, quelle que soit la forme cristallographique (par exemple feuillet, cubique, hexagonale, orthorhombique, etc.). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydrocarbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

La composition peut comprendre en outre un polymère additionnel tel qu'un polymère filmogène. Selon la présente invention, on entend par "polymère filmogène", un polymère apte à former à lui seul ou en présence d'un agent auxiliaire de filmification, un film continu et adhérent sur un support, notamment sur les matières kératiniques. Parmi les polymères filmogènes susceptibles d'être utilisés dans la composition de la présente invention, on peut citer les polymères synthétiques, de type radicalaire ou de type polycondensat, les polymères d'origine naturelle et leurs mélanges, en particulier les polymères acryliques, les polyuréthanes, les polyesters, les polyamides, les polyurées, les polymères cellulosiques comme la nitrocellulose.

La composition selon l'invention peut également comprendre des ingrédients couramment utilisés en cosmétique, tels que les vitamines, les épaississants, les gélifiants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents antipelliculaires, les agents propulseurs, les céramides, ou leurs mélanges. Bien entendu, l'homme du métier veillera à choisir ce ou ces éventuels composés complémentaires, et/ou leur quantité, de manière telle que les propriétés avantageuses de la composition selon l'invention ne soient pas, ou substantiellement pas, altérées par l'adjonction envisagée.

La composition selon l'invention peut se présenter notamment sous forme de suspension, de dispersion, de solution notamment organique, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de stick (bâton).

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Les dispersions selon l'invention sont donc au final formées de particules polymériques souples, monodisperses en taille, de diamètre assez grand (supérieur de préférence à 100 nm), et vont conduire à des dépôts filmogènes, souples et confortables, ainsi que brillants, à la température d'observation (25°C), intéressants notamment pour les applications maquillage, coloration capillaire, après-shampoings, soin de la peau et/ou solaire.

De plus, ladite dispersion étant en milieu huileux, il devient aisé de la formuler dans des compositions cosmétiques à base de milieu huileux couramment utilisé en cosmétique, en particulier les milieux anhydres.

La composition selon l'invention peut être une composition de maquillage, notamment un produit pour le teint tel qu'un fond de teint, un fard à joues ou à paupières; un produit pour les lèvres tel qu'un rouge à lèvres, un soin des lèvres, un brillant à lèvres (gloss); un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux).

La composition selon l'invention peut être une composition de protection ou de soin de la peau du visage, du cou, des mains ou du corps, notamment une composition antirides, antifatigue permettant de donner un coup d'éclat à la peau, une composition hydratante ou traitante; une composition antisolaire ou de bronzage artificiel.

La composition selon l'invention peut être également un produit capillaire, notamment pour le maintien de la coiffure ou la mise en forme des cheveux; le soin, le conditionnement ou l'hygiène des cheveux; voir la coloration des cheveux. Les compositions capillaires sont de préférence des shampooings, des après-shampoings, des gels, des lotions de mise en plis, des lotions pour le brushing, des compositions de fixation et de coiffage telles que les laques ou spray. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray,

une mousse pour la fixation ou le traitement des cheveux.

La composition selon l'invention trouve une application toute particulière comme composition de maquillage, notamment de rouge à lèvres, de gloss (brillant à lèvres), de fard à paupières, de fard à joues, de vernis à ongles ou de soin des ongles, ainsi que de soin des cheveux.

L'invention a aussi pour objet un procédé de traitement cosmétique, notamment de maquillage, de nettoyage, de protection solaire, de mise en forme, de coloration, ou de soin des matières kératiniques, notamment de la peau du corps ou du visage, des lèvres, des ongles, des cheveux et/ou des cils, comprenant l'application sur lesdites matières d'une composition cosmétique telle que définie précédemment.

L'invention est illustrée plus en détails dans les exemples suivants, donnés à titre d'illustration.

**Exemple 1 : préparation de la dispersion D1**

A/

On mélange dans un ballon tricol de 100 ml, connecté à un réfrigérant muni d'un bulleur, 30 g (4,78 mol/l) d'acrylate de 2-éthylhexyle, 4,6.$10^{-3}$ mol/l d'amorceur (Trigonox 21S d'Akzo), 4,4.$10^{-2}$ mol/l d'agent de contrôle (TTC ou S,S-bis[1-(2-éthylhexyloxycarbonyl)éthyt]trithiocarbonate).

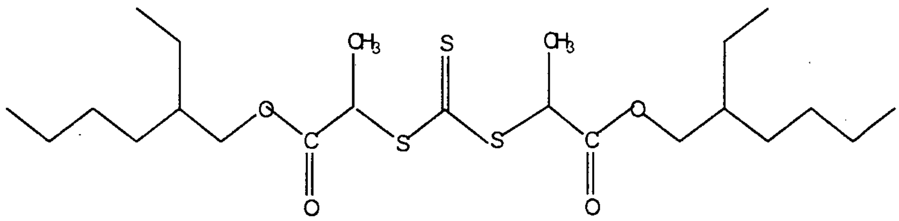

structure du TTC

Le tricol est fermé puis l'oxygène est évacué par un bullage d'azote pendant 30 minutes. Le ballon est ensuite introduit dans un bain d'huile thermostaté à 60°C, sous une agitation de 250 à 300 tr/min. La réaction est arrêtée après 5 heures. Le polymère est précipité deux fois à froid dans le méthanol puis est séché sous pression réduite pendant 24 heures à 25°C.

Les caractéristiques finales du polymère sont présentées ci-dessous :

| Taux de conversion (%) | $Mn^{PS}$ * | Ip ** | $Mn^{LS}$ *** | DP **** |
|---|---|---|---|---|
| 89 | 16 400 | 1,08 | 19 200 | 102 |

* $Mn^{PS}$: masse molaire moyenne en nombre déterminée par GPC avec une calibration polystyrène
** Ip =Mw/Mn; Mw et Mn étant les masses molaires moyenne en poids et en nombre, déterminées par GPC avec une calibration polystyrène ($Mn^{PS}$ et $Mw^{PS}$)
*** $Mn^{LS}$ : masse molaire moyenne en nombre, déterminée par GPC avec une détection par diffusion de lumière.
**** DP : degré de polymérisation déterminé à partir de la $Mn^{LS}$

B/

Dans un tricol de 100ml, on dispose :

- 2,0 mol/l d'acrylate de méthyle (2 mole par litre d'isododécane),
- le polymère préparé ci-dessus, en une quantité telle que le rapport en poids polymère préparé ci-dessus/acrylate de méthyle est de 1,5/100
- 1,33.$10^{-3}$ mol/l d'amorceur (Trigonox 21 S d'Akzo),
- de l'isododécane de manière à obtenir un taux de matière sèche de 22,3% en poids.

Le mélange réactionnel est soumis à un bullage d'azote pendant 30 minutes, sous agitation; puis est plongé dans un

bain d'huile préchauffé à 80°C, sous une agitation de 250 tr/min. L'apparition d'un trouble correspond au démarrage de la réaction. Le tricol est retiré après 4 heures de réaction, et passé sous eau froide pour arrêter la réaction.

On obtient au final, une dispersion, dans l'isododécane, de polyacrylate de méthyle, stabilisé par un polymère tribloc poly(acrylate d'éthyle 2-hexyle)-b-polyacrylate de méthyle-b-poly(acrylate d'éthyle 2-hexyle), dont les caractéristiques sont les suivantes :

| Taux de conversion (%) | Extrait sec | Diamètre [b] (nm) | PDI [c] |
|---|---|---|---|
| 77% | 17% | 319 | 0,03 |
| [b] : diamètre moyen déterminé par diffusion dynamique de lumière (Malvern Zetasizer Nano S90)<br>[c] : indice de polydispersité en taille des particules. | | | |

**Exemple 2 : préparation de la dispersion D2**

Dans un tricol de 250 ml, on dispose :

- 2,01 mol/l d'acrylate de méthyle (2,01 mole par litre d'isododécane),
- le polymère préparé à l'étape A de l'exemple 1 ci-dessus, avec un rapport en poids polymère /acrylate de méthyle de 3,01/100,
- $1{,}53.10^{-3}$ mol/l d'amorceur (Trigonox 21 S d'Akzo),
- de l'isododécane de manière à obtenir un taux de matière sèche de 22,7% en poids.

Le mélange réactionnel est soumis à un bullage d'azote pendant 30 minutes, sous agitation; puis est plongé dans un bain d'huile préchauffé à 80°C, sous une agitation de 250 tr/min. L'apparition d'un trouble correspond au démarrage de la réaction. Le tricol est retiré après 6 heures de réaction, et passé sous eau froide pour arrêter la réaction.

On obtient au final, une dispersion, dans l'isododécane, de polyacrylate de méthyle, stabilisé par un polymère tribloc poly(acrylate d'éthyle 2-hexyle)-b-polyacrylate de méthyle-b-poly(acrylate d'éthyle 2-hexyle), dont les caractéristiques sont les suivantes :

| Taux de conversion (%) | Extrait sec | Diamètre [b] (nm) | PDI [c] |
|---|---|---|---|
| 94% | 21% | 212 | 0,02 |

Par DMTA, on ne constate qu'une seule Tg pour la dispersion (Tg=38°C), correspondant à celle du PAMe; on ne perçoit pas la Tg basse (~-50°C en théorie) de la partie PA2EH.
La DMTA a été réalisée après 6 jours de séchage (25°C et 45% HR) de films avec une épaisseur de l'ordre de 250 µm au final.
Appareil : DMA 2980 (TA Instruments)
DMTA : de -100 à +200°C à 3°C/ min
On ne perçoit pas de collant dans le film au final.

**Exemple 3 : préparation de la dispersion D3**

Dans un tricol de 250 ml, on dispose :

- 2,0 mol/l d'acrylate de méthyle (2,0 mole par litre d'isododécane),
- le polymère préparé à l'étape A de l'exemple 1 ci-dessus, avec un rapport en poids polymère /acrylate de méthyle de 6,05/100,
- $1{,}23.10^{-3}$ mol/l d'amorceur (Trigonox 21 S d'Akzo),
- de l'isododécane de manière à obtenir un taux de matière sèche de 23,2% en poids.

Le mélange réactionnel est soumis à un bullage d'azote pendant 30 minutes, sous agitation; puis est plongé dans un bain d'huile préchauffé à 80°C, sous une agitation de 250 tr/min. L'apparition d'un trouble correspond au démarrage de la réaction. Le tricol est retiré après 6 heures de réaction, et passé sous eau froide pour arrêter la réaction.

On obtient au final, une dispersion, dans l'isododécane, de polyacrylate de méthyle, stabilisé par un polymère tribloc poly(acrylate d'éthyle 2-hexyle)-b-polyacrylate de méthyle-b-poly(acrylate d'éthyle 2-hexyle), dont les caractéristiques sont les suivantes :

| Taux de conversion (%) | Extrait sec | Diamètre [b] (nm) | PDI [c] |
|---|---|---|---|
| 97% | 23% | 117 | 0,03 |

Par DMTA, on ne constate qu'une seule Tg pour la dispersion (Tg=36°C).
On ne perçoit pas de collant dans le film au final.

**Exemple 4 : préparation de la dispersion D4**

Dans un tricol de 250 ml, on dispose :

- 1,93 mol/l d'acrylate de méthyle (1,93 mole par litre d'isododécane),
- le polymère préparé à l'étape A de l'exemple 1 ci-dessus, avec un rapport en poids polymère /acrylate de méthyle de 2,93/100,
- $1{,}51.10^{-3}$ mol/l d'amorceur (Trigonox 21 S d'Akzo),
- de l'isododécane de manière à obtenir un taux de matière sèche de 22,3% en poids.

Le mélange réactionnel est soumis à un bullage d'azote pendant 30 minutes, sous agitation; puis est plongé dans un bain d'huile préchauffé à 80°C, sous une agitation de 250 tr/min. L'apparition d'un trouble correspond au démarrage de la réaction. Après 1 heure et 10 minutes de réaction, on ajoute 1% molaire de réticulant par rapport à l'acrylate de méthyle (EGDMA : éthylène glycol diméthacrylate), et on continue la réaction. Le tricol est retiré après 6 heures de réaction au total, et passé sous eau froide pour arrêter la réaction.

On obtient au final, une dispersion, dans l'isododécane, de polyacrylate de méthyle, stabilisé par un polymère tribloc poly(acrylate d'éthyle 2-hexyle)-b-polyacrylate de méthyle-b-poly(acrylate d'éthyle 2-hexyle), dont les caractéristiques sont les suivantes :

| Taux de conversion (%) | Extrait sec | Diamètre [b] (nm) | PDI [c] |
|---|---|---|---|
| 91% | Environ 20% | 208 | 0,02 |

Par DMTA, on ne constate qu'une seule Tg pour la dispersion (Tg=39°C).
On ne perçoit pas de collant dans le film au final.

**Exemple 5 : préparation de la dispersion comparative (hors invention)**

Dans un réacteur, on charge 600 g d'isododécane et 230 g d'heptane, puis on introduit en pluie 30 g de Kraton G1701 E (copolymère bloc styrène/isoprène) sous agitation. Après 30 minutes d'agitation, on ajoute 50 g d'acrylate de méthyle, 10 g d'acide acrylique et 3 g d'amorceur (Trigonox 21 S). On porte le milieu réactionnel à 90°C en 45 minutes; on note un blanchiment dès l'arrivée à 90°C avec une exothermie de 10°C. A la fin de l'exothermie (environ 15 min après le blanchiment), on coule en 1 heure et à 90°C, 140 g d'acrylate de méthyle et 2,2 g d'amorceur (Trigonox 21 S). On laisse 3 heures à 90°C, puis on distille l'heptane et on le remplace par 200 g d'isododécane.
On obtient une dispersion de particules d'acrylate de méthyle et d'acide acrylique, stabilisées en surface par un copolymère bloc styrène/isoprène, dans l'isododécane.

**Exemple 6 : Caractérisations des dispersions**

On mesure la brillance et on compare le collant en présence de sébum ou d'huile d'olive, pour les dispersions D2, D3 et D4 de l'invention en comparaison avec la dispersion de l'état de l'art, qui est elle-même brillante et de bonne tenue, résistante aux agressions (huile, sébum), mais qui est stabilisée par un polymère lié de manière non covalente par le biais d'interactions physiques sur les particules de polymère, telle que décrite dans EP749747.

Brillance mesurée au brillancemètre sur un dépôt sec de polymère

La brillance est mesurée à l'aide d'un brillancemètre, de manière conventionnelle par la méthode suivante : sur une carte de contraste de marque LENETA et de référence FORM 1A PENOPAC, on étale une couche de 200 μm

d'épaisseur d'une dispersion du polymère à 50% dans l'isododécane à l'aide d'un étaleur automatique. La couche recouvre au moins le fond noir de la carte. On laisse sécher le dépôt 24 heures à une température de 25°C, puis on procède à la mesure de la brillance à 20° sur le fond noir à l'aide d'un brillancemètre de marque DR LANGE, REF03. Une mesure à 20° supérieure à environ 50 donne une brillance jugée acceptable, et très satisfaisante si la mesure est supérieure à 60.

On obtient les résultats suivants :

| | D2 | D3 | D4 | comparatif |
|---|---|---|---|---|
| Brillance 20° | 50 | 62 | 62 | 52 |

<u>Collant</u>

La sensibilité des films au sébum et à l'huile d'olive a été évaluée de façon sensorielle, de la manière suivante : on dépose un film d'environ 20 μm d'épaisseur sur une surface plane, à partir d'une dispersion à 50% de matière sèche de polymère, et on lèche sécher 3 jours de séchage à 25°C ; on dépose ensuite 1 goutte de sébum ou 1 goutte d'huile d'olive sur le film, on laisse en contact 5 minutes ou 1 heure, puis on apprécie le collant du film au doigt, de manière comparative.

On obtient les résultats suivants :

| | D2 | D3 | D4 |
|---|---|---|---|
| Collant huile olive après 5 minutes | Identique au comparatif | Identique au comparatif | Identique au comparatif |
| Collant sébum après 5 minutes | Identique au comparatif | Identique au comparatif | Identique au comparatif |
| Collant huile olive après 1 heure | Identique au comparatif | Identique au comparatif | Identique au comparatif |
| Collant sébum après 1 heure | Identique au comparatif | Identique au comparatif | Identique au comparatif |

On constate que les dispersions selon l'invention donnent des films au moins aussi brillants, voir plus brillants, que ceux obtenus avec l'état de l'art ; les films selon l'invention ne développent pas de collant en présence d'huile d'olive ou de sébum liquide, que cela soit après 5 minutes ou 1 heure de contact.
On peut de plus penser que les dispersions selon l'invention seront plus stables dans le temps que celle de l'état de l'art.

**<u>Exemple 7 : Composition de mascara</u>**

On prépare un mascara ayant la composition suivante :

| | |
|---|---|
| Hectorite modifiée (Bentone® 38V d'Elementis) | 2 g |
| pigments | 2 g |
| Dispersion D1 | 15 g MS* |
| Isododécane | qsp 100 g |

*MS : matière sèche

**<u>Exemple 8 : Stick de rouge à lèvres</u>**

On prépare la composition de rouge à lèvres suivante (% en poids):

- Cire de polyéthylène 3%
- Dispersion D2 13% en MS
- Isododécane qsp 100%

La composition obtenue présente de bonnes propriétés cosmétiques.

**<u>Exemple 9 : Gloss pour les lèvres</u>**

On prépare un gloss ayant la composition suivante (% en poids) :

- Dispersion D3 5% en MS
- Parléam qsp 100%

La composition obtenue présente de bonnes propriétés cosmétiques.

**Exemple 10 : Fond de teint E/H**

On prépare une composition de fond de teint comprenant les composés suivants :

*Phase A*

| | |
|---|---|
| Cétyl Diméthicone copolyol (ABIL EM 90 de la société GOLDSCHMIDT) | 3% |
| Succinate d'isostéaryl diglycéryle (IMWITOR 780K de la société CONDEA) | 0,6% |
| Pigments (oxydes de fer et de titane hydrophobes) | 1 % |
| Dispersion D3 | 20% (soit 1,3% MS) |
| Isododécane | qsp 100 g (sur la phase A) |

*Phase B*

| | |
|---|---|
| Sulfate de magnésium | 0,7 g |
| Conservateur | q.s. |
| Eau | qsp 100 g (sur la phase B) |

La composition obtenue présente de bonnes propriétés cosmétiques.

**Exemple 11 : Vernis à ongles**

On prépare un vernis à ongles ayant la composition suivante :

- Dispersion D1 4 g de MS
- Acétate de Butyle 25 g
- Isopropanol 11 g
- Hexylène Glycol 2,5 g
- pigment 1 g
- Hectorite modifiée (Bentone® 27V d'Elementis) 1,3 g
- Acétate d'éthyle qsp 100 g

Ce vernis a été jugé comme présentant de très bonnes propriétés cosmétiques.

**Exemple 12 : poudre**

On prépare une poudre compactée ayant la composition suivante :

*Composition A :*

- Talc 10 g
- Oxychlorure de bismuth 10 g
- Stéarate de zinc 4 g
- Dispersion D2 5 g MS

*Composition B :*

- Oxydes de fer 2 g
- Huile de vaseline 6 g

La poudre est obtenue de la façon suivante: on broie la composition A dans un broyeur de type KENWOOD pendant environ 5 minutes sous faible agitation, on ajoute la composition B et on broie l'ensemble environ 2 minutes à la même vitesse, puis 3 minutes à une vitesse plus rapide. On tamise ensuite la préparation sur un tamis de 0,16 mm, puis on compacte ce mélange dans des coupelles. On obtient une poudre compactée présentant de bonnes propriétés cosmétiques.

**Exemple 13 : gel pour le visage**

On prépare la composition suivante :

- palmitate d'isopropyle 10 g
- hectorite modifiée 0,15 g
- septaoléate de sorbitane oxyéthyléné (40OE) 5 g
- dispersion D2 9 g MS
- Parléam qsp 100 g

On obtient un gel ayant de bonnes propriétés cosmétiques.

**Exemple 14 : huile de soin**

On prépare la composition suivante :

- dispersion D2 12 g MS
- huile de jojoba 10 g
- huile de soja 10 g

On obtient une huile de soin qui peut être appliquée sur le corps ou le visage.

## Revendications

**1.** Dispersion de particules de polymère, dans un milieu carboné liquide, lesdites particules présentant un coeur polymérique souple étant susceptibles d'être obtenues par polymérisation :

i) de 60 à 100% en poids, par rapport au poids total de monomères, de monomères dont l'homopolymère a une Tg strictement inférieure à 20°C choisis parmi, seul ou en mélange, les monomères suivants, ainsi que leurs sels :

- les (méth)acrylates de formule $CH_2=C(CH_3)-COOR$ ou $CH_2=CH-COOR$, dans laquelle R représente un groupe alkyle, linéaire, ramifié ou cyclique, saturé ou insaturé, voire aromatique, comprenant 1 à 32 atomes de carbone, pouvant comporter un ou plusieurs substituants choisis parmi -OH, les atomes d'halogène et -NR'R" avec R' et R", identiques ou différents, choisis parmi les alkyles, linéaires ou ramifiés, en C1-C4; et/ou interrompu par un atome d'oxygène ;
- les monomères éthyléniques dont le groupement ester contient des silanes, des silsesquioxanes, des siloxanes, des carbosiloxanes dendrimères, à l'exception des monomères ne contenant qu'un atome de silicium ;

ii) et éventuellement de 0,1 à 40% en poids de monomères dont l'homopolymère a une Tg supérieure ou égale à 20°C ;
la température de transition vitreuse (Tg) du polymère en résultant, formant le coeur souple, étant strictement inférieure à 20°C ;

, et lesdites particules étant stabilisées en surface par un polymère stabilisant séquencé comprenant au moins une séquence soluble dans ledit milieu carboné et au moins une séquence insoluble dans ledit milieu carboné; ladite séquence insoluble comprenant 50 à 100% en poids de monomère insoluble dans ledit milieu, choisi parmi, seul ou en mélange, les monomères suivants ainsi que leurs sels:

-(i) les (méth)acrylates de formule : $CH_2=C(CH_3)-COOR'_1$ ou $CH_2=CH-COOR'_1$ dans laquelle R'1 représente un groupe choisi parmi :

- un groupe alkyle, linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène et -NR'R" avec R' et R", identiques ou différents, choisis parmi les alkyles, linéaires ou ramifiés, en C1-C4; et/ou pouvant être substitué par au moins un groupe polyoxyalkylène, ledit groupe polyoxyalkylène étant constitué par la répétition de 5 à 30 motifs oxyalkylène; sont exclus de cette définition le méthacrylate de tertiobutyle et l'acrylate d'isobutyle.
- un groupe alkyle cyclique comprenant de 3 à 6 atomes de carbone, ledit groupe pouvant comporter dans sa chaîne un ou plusieurs hétéroatomes choisis parmi O, N et S, et/ou pouvant comporter un ou plusieurs substituants choisis parmi OH et les atomes d'halogène;

-(ii) les (méth)acrylamides de formule : $CH_2=C(CH_3)-CONR'_3R'_4$ ou $CH_2=CH-CONR'_3R'_4$, dans laquelle :

- R'3 et R'4, identiques ou différents, représentent un atome d'hydrogène ou un groupe alkyle, linéaire ou ramifié, comportant de 1 à 6 atomes de carbone, pouvant comporter un ou plusieurs substituants choisis parmi -OH, les atomes d'halogène et -NR'R" avec R' et R", identiques ou différents, choisis parmi les alkyles, linéaires ou ramifiés, en C1-C4; ou
- R'3 représente un atome d'hydrogène et R'4 représente un groupe 1,1-diméthyl3-oxobutyle ;

-(iii) les monomères à insaturation(s) éthylénique(s) comprenant au moins une fonction acide carboxylique, phosphorique ou sulfonique, et leurs sels;
-(iv) les esters de vinyle de formule $R'6-COO-CH=CH_2$ dans laquelle R'6 représente un groupe alkyle linéaire ou ramifié, comprenant de 1 à 6 atomes, ou un groupe alkyle cyclique comportant de 3 à 6 atomes de carbone et/ou un groupe aromatique;
-(v) les monomères à insaturation(s) éthylénique(s) comprenant au moins une fonction amine tertiaire;
-(vi) les di-n-alkylitaconates de formule $CH_2=C(CH_2-COO(CH_2)_{n-1}-CH_3)-COO(CH_2)_{n-1}-CH_3$, avec n étant un entier de 0 à 4;
-(vii) les monomères éthyléniques dont le groupement ester contient des silanes, des silsesquioxanes, des siloxanes, des carbosiloxanes dendrimères, à l'exception des monomères ne contenant qu'un atome de silicium.
-(viii) les macromonomères PDMS tels que les polydiméthylsiloxanes à groupement terminal monoacryloyloxy ou monométhacryloyloxy, et notamment eux de formule suivante :

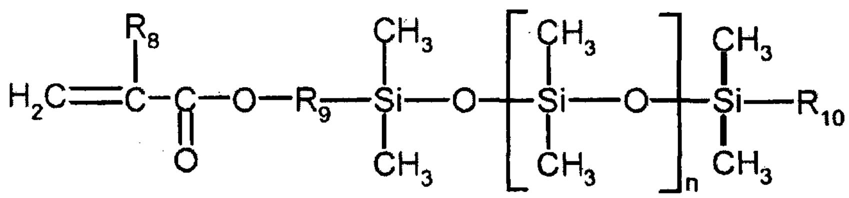

dans laquelle :

- R8 désigne un atome d'hydrogène ou un groupement méthyle;
- R9 désigne un groupe hydrocarboné divalent, linéaire ou ramifié, ayant de 1 à 10 atomes de carbone et contenant éventuellement une ou deux liaisons éther -O- ;
- R10 désigne un groupe alkyle linéaire ou ramifié, ayant de 1 à 10 atomes de carbone ;
- n désigne un nombre entier allant de 1 à 300

**2.** Dispersion selon la revendication précédente, dans laquelle le polymère stabilisant séquencé comprend trois séquences (tribloc), notamment du type tribloc soluble/insoluble/soluble.

**3.** Dispersion selon l'une des revendications précédentes, dans laquelle la séquence soluble du polymère stabilisant séquencé comprend 50 à 100% en poids de monomère soluble dans ledit milieu, choisi parmi, seul ou en mélange, les monomères suivants :

- les méthacrylates de formule $CH_2 = C(CH_3)\text{-}COOR_1$
dans laquelle $R_1$ représente un groupe alkyle linéaire ou ramifié en C8-C22; ou un groupe alkyle cyclique ayant 8 à 30 atomes de carbone; ou le groupe tertiobutyle;
- les acrylates de formule $CH_2 = CH\text{-}COOR_2$
dans laquelle $R_2$ représente un groupe alkyle linéaire ou ramifié en C8-C22; ou un groupe alkyle cyclique ayant 8 à 30 atomes de carbone; ou le groupe isobutyle ;
- les (méth)acrylamides de formule $CH_2{=}C(CH_3)\text{-}CONR_3R_4$ ou $CH_2{=}CH\text{-}CONR_3R_4$, dans lesquelles $R_3$ représente un atome d'hydrogène ou un groupe alkyle linéaire ou ramifié en C1-C12, et $R_4$ représente un groupe alkyle en $C_8$ à $C_{12}$ linéaire ou ramifié;
- les di-n-alkylitaconates de formule $CH_2{=}C(CH_2\text{-}COO(CH_2)_{n-1}\text{-}CH_3)\text{-}COO(CH_2)_{n-1}\text{-}CH_3$, avec n étant un entier supérieur ou égal à 5;
- les esters de vinyle de formule $R_5\text{-}CO\text{-}O\text{-}CH{=}CH_2$ où $R_5$ représente un groupe alkyle en $C_8$ à $C_{22}$ linéaire ou ramifié ;
- les éthers d'alcool vinylique et d'alcool de formule $R_6O\text{-}CH{=}CH_2$ dans laquelle $R_6$ représente un groupe alkyle linéaire ou ramifié, comprenant de 8 à 22 atomes de carbone;
- les monomères éthyléniques dont le groupement ester contient des silanes ou des siloxanes, et ne contenant qu'un atome de silicium tel que le (méth)acryloxypropyl trimethoxysilane;
- des macromonomères carbonés ayant un groupe terminal polymérisable.

**4.** Dispersion selon l'une des revendications précédentes, dans laquelle la séquence insoluble du polymère stabilisant séquencé comprend 50 à 100% en poids de monomère insoluble dans ledit milieu, choisi parmi, seul ou en mélange, les monomères suivants ainsi que leurs sels:

- les (méth)acrylates de formule $CH_2{=}C(CH_3)\text{-}COOR'_1$ ou $CH_2{=}CH\text{-}COOR'_1$ ; dans laquelle R'1 représente un groupe alkyle, linéaire ou ramifié, comprenant de 1 à 6 atomes de carbone, ledit groupe pouvant comporter dans sa chaîne un ou plusieurs hétéroatomes choisis parmi O, N et S; et/ou pouvant comporter un ou plusieurs substituants choisis parmi -OH, les atomes d'halogène et -NR'R" avec R' et R", identiques ou différents, choisis parmi les alkyles, linéaires ou ramifiés, en C1-C4;
- les monomères à insaturation(s) éthylénique(s) comprenant au moins une fonction acide carboxylique et leurs sels;
- les monomères éthyléniques dont le groupement ester contient des silanes;
- les polydiméthylsiloxanes à groupement terminal monoacryloyloxy ou monométhacryloyloxy, de formule suivante :

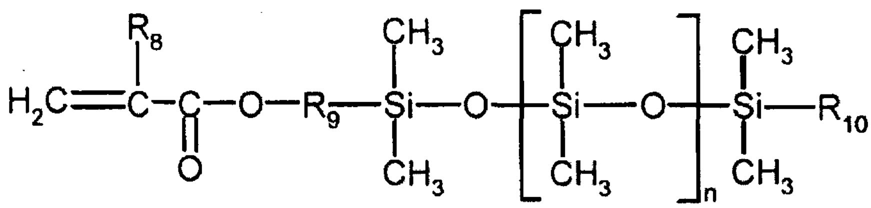

dans laquelle :

- R8 désigne un atome d'hydrogène ou un groupement méthyle;
- R9 désigne un groupe hydrocarboné divalent, linéaire ou ramifié, ayant de 1 à 10 atomes de carbone et contenant éventuellement une ou deux liaisons éther -O- ;
- R10 désigne un groupe alkyle linéaire ou ramifié, ayant de 1 à 10 atomes de carbone,
- n désigne un nombre entier allant de 1 à 300.

**5.** Dispersion selon l'une des revendications précédentes, dans laquelle le milieu carboné liquide comprend au moins 50% en poids, par rapport au poids total du milieu carboné, de composé carboné, liquide à 25°C, ayant un paramètre

de solubilité global selon l'espace de solubilité de HANSEN inférieur ou égal à 20 $(MPa)^{1/2}$, ou un mélange de tels composés.

**6.** Dispersion selon l'une des revendications précédentes, dans laquelle le milieu carboné liquide comprend au moins un composé carboné choisi parmi :

- les huiles végétales formées par des esters d'acides gras et de polyols, en particulier les triglycérides,
- les esters de formule RCOOR' dans laquelle R représente le reste d'un acide gras supérieur comportant 7 à 19 atomes de carbone et R' représente une chaîne hydrocarbonée comportant de 3 à 20 atomes de carbone,
- les alcanes linéaires ou ramifiés en C8-C60, volatils ou non volatils;
- les alcanes cycliques, non aromatiques, en C5-C12; volatils ou non volatils;
- les éthers ayant 7 à 30 atomes de carbone;
- les cétones ayant 8 à 30 atomes de carbone;
- les monoalcools gras aliphatiques ayant 12 à 30 atomes de carbone, la chaîne hydrocarbonée ne comportant pas de groupement de substitution,
- leurs mélanges.

**7.** Dispersion selon l'une des revendications précédentes, dans laquelle le milieu carboné liquide comprend au moins du myristate d'isopropyle, de l'octyldodécanol, des isoparaffines en C5-C60, de l'isododécane, de l'isohexadécane, de l'isononanoate d'isononyle, du Parléam.

**8.** Dispersion selon l'une des revendications précédentes, présentant un taux de matière sèche de 5 à 40% en poids, notamment de 6 à 30% en poids, voire de 7 à 25% en poids.

**9.** Composition cosmétique comprenant, dans un milieu cosmétiquement acceptable, au moins une dispersion selon l'une des revendications 1 à 8.

**10.** Composition selon la revendication 9, dans laquelle la dispersion est présente en une quantité telle que la quantité de matière sèche (ou matière active) de polymère dans la composition représente 1 à 70% en poids, de préférence 2 à 50% en poids, notamment 5 à 40% en poids, voire 7 à 30% en poids, par rapport au poids total de la composition.

**11.** Composition selon l'une des revendications 9 à 10, comprenant au moins un ingrédient choisi parmi les huiles, les solvants, les corps gras solides à température ambiante, les corps gras pâteux, les gommes; de l'eau; les matières colorantes; les charges; les polymères additionnels; les vitamines, les épaississants, les gélifiants, les oligo-éléments, les adoucissants, les séquestrants, les parfums, les agents alcalinisants ou acidifiants, les conservateurs, les filtres solaires, les tensioactifs, les antioxydants, les agents anti-chutes des cheveux, les agents antipelliculaires, les agents propulseurs, les céramides, ou leurs mélanges.

**12.** Composition selon l'une des revendications 9 à 11, se présentant sous forme d'une composition de maquillage, notamment un produit pour le teint tel qu'un fond de teint, un fard à joues ou à paupières; un produit pour les lèvres tel qu'un rouge à lèvres, un soin des lèvres, un brillant à lèvres (gloss); un produit anti-cernes; un blush, un mascara, un eye-liner; un produit de maquillage des sourcils, un crayon à lèvres ou à yeux; un produit pour les ongles tel qu'un vernis à ongles ou un soin des ongles; un produit de maquillage du corps; un produit de maquillage des cheveux (mascara ou laque pour cheveux) ; une composition de protection ou de soin de la peau du visage, du cou, des mains ou du corps, notamment une composition antirides, antifatigue permettant de donner un coup d'éclat à la peau, une composition hydratante ou traitante; une composition antisolaire ou de bronzage artificiel; un produit capillaire, notamment pour le maintien de la coiffure ou la mise en forme des cheveux; le soin, le conditionnement ou l'hygiène des cheveux; voir la coloration des cheveux.

**13.** Procédé de traitement cosmétique, notamment de maquillage, de nettoyage, de protection solaire, de mise en forme, de coloration, ou de soin des matières kératiniques, notamment de la peau du corps ou du visage, des lèvres, des ongles, des cheveux et/ou des cils, comprenant l'application sur lesdites matières d'une composition cosmétique selon l'une des revendications 9 à 12.

## Patentansprüche

**1.** Dispersion von Polymerteilchen in einem flüssigen Medium auf Basis von Kohlenstoff, wobei die Teilchen einen

flexiblen Polymerkern aufweisen und durch Polymerisation von:

i) 60 bis 100 Gew.-%, bezogen auf das Gesamtgewicht der Monomere, Monomeren, deren Homopolymer eine Tg von streng weniger als 20°C aufweist und die alleine oder als Mischung aus den folgenden Monomeren sowie deren Salzen ausgewählt sind:

- (Meth)acrylaten der Formel $CH_2=C(CH_3)-COOR$ bzw. $CH_2=CH-COOR$, worin R für eine lineare, verzweigte oder cyclische, gesättigte oder ungesättigte oder sogar aromatische Alkylgruppe mit 1 bis 32 Kohlenstoffatomen steht, die einen oder mehrere Substituenten enthalten kann, die aus -OH, Halogenatomen und -NR'R" ausgewählt sind, wobei R' und R" gleich oder verschieden sind und aus linearen oder verzweigten $C_1$-$C_4$-Alkylgruppen ausgewählt sind; und/oder durch ein Sauerstoffatom unterbrochen sein kann;
- ethylenischen Monomeren, deren Estergruppe Silane, Silsesquioxane, Siloxane oder Carbosiloxan-Dendrimere enthält, mit Ausnahme von Monomeren, die nur ein Siliciumatom enthalten;

ii) und gegebenenfalls 0,1 bis 40 Gew.-% Monomeren, deren Homopolymer eine Tg größer gleich 20°C aufweist; erhältlich sind;
wobei die Glasübergangstemperatur (Tg) des sich daraus ergebenden, den flexiblen Kern bildenden Polymers streng unter 20°C liegt;
und wobei die Teilchen durch ein stabilisierend wirkendes Blockpolymer mit mindestens einem Block, der in dem Medium auf Basis von Kohlenstoff löslich ist, und mindestens einem Block, der in dem Medium auf Basis von Kohlenstoff unlöslich ist, oberflächenstabilisiert sind; wobei der unlösliche Block 50 bis 100 Gew.-% in dem Medium unlösliches Monomer umfasst, das alleine oder als Mischung aus den folgenden Monomeren sowie deren Salzen ausgewählt ist:

- (i) (Meth)acrylaten der Formel: $CH_2=C(CH_3)-COOR'_1$ bzw. $CH_2=CH-COOR'_1$,
worin $R'_1$ für eine aus folgenden Gruppen ausgewählte Gruppe steht:

- eine lineare oder verzweigte Alkylgruppe mit 1 bis 6 Kohlenstoffatomen, wobei die Gruppe in ihrer Kette ein oder mehrere aus O, N und S ausgewählte Heteroatome enthalten kann und/oder einen oder mehrere Substituenten enthalten kann, die aus -OH, Halogenatomen und -NR'R " ausgewählt sind, wobei R' und R" gleich oder verschieden sind und aus linearen oder verzweigten $C_1$-$C_4$-Alkylgruppen ausgewählt sind; und/oder durch mindestens eine Polyoxyalkylengruppe substituiert sein kann, wobei die Polyoxyalkylengruppe aus einer Wiederholung von 5 bis 30 Oxyalkylen-Einheiten besteht; aus dieser Definition sind tert.-Butylmethacrylat und Isobutylacrylat ausgeschlossen;
- eine cyclische Alkylgruppe mit 3 bis 6 Kohlenstoffatomen, wobei die Gruppe in ihrer Kette ein oder mehrere aus O, N und S ausgewählte Heteroatome enthalten kann und/oder einen oder mehrere Substituenten enthalten kann, die aus OH und Halogenatomen ausgewählt sind;

- (ii) (Meth) acrylamiden der Formel: $CH_2=C(CH_3)-CONR'_3R'4$ bzw. $CH_2=CH-CONR'_3R'_4$, worin:

$R'_3$ und $R'_4$ gleich oder verschieden sind und für ein Wasserstoffatom oder eine lineare oder verzweigte Alkylgruppe mit 1 bis 6 Kohlenstoffatomen, die einen oder mehrere Substituenten enthalten kann, die aus -OH, Halogenatomen und -NR'R" ausgewählt sind, wobei R' und R" gleich oder verschieden sind und aus linearen oder verzweigten $C_1$-$C_4$-Alkylgruppen ausgewählt sind, stehen; oder

- $R'_3$ für ein Wasserstoffatom steht und $R'_4$ für eine 1,1-Dimethyl-3-oxobutylgruppe steht;

- (iii) Monomeren mit einer oder mehreren ethylenischen Ungesättigtheiten und mindestens einer Carbonsäure-, Phosphorsäure- oder Sulfonsäurefunktion und deren Salzen;
- (iv) Vinylestern der Formel $R'_6-COO-CH=CH_2$, worin $R'_6$ für eine lineare oder verzweigte Alkylgruppe mit 1 bis 6 Atomen oder eine cyclische Alkylgruppe mit 3 bis 6 Kohlenstoffatomen und/oder einer aromatischen Gruppe steht;
- (v) Monomeren mit einer oder mehreren ethylenischen Ungesättigtheiten und mindestens einer tertiären Aminfunktion;
- (vi) Di-n-alkylitaconaten der Formel $CH_2=C(CH_2-COO(CH_2)_{n-1}-CH_3)-COO(CH_2)_{n-1}-CH_3$, wobei n für eine ganze Zahl von 0 bis 4 steht;
- (vii) ethylenischen Monomeren, deren Estergruppe Silane, Silsesquioxane, Siloxane oder Carbosiloxan-Dendrimere enthält, mit Ausnahme von Monomeren, die nur ein Siliciumatom enthalten;

- (viii) PDMS-Makromonomeren wie Polydimethylsiloxanen mit einer Monoacryloyloxy- oder Monomethacryloyloxy-Endgruppe, und insbesondere denjenigen der folgenden Formel:

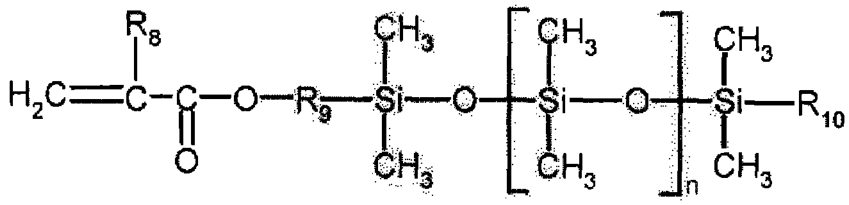

worin:

- $R_8$ für ein Wasserstoffatom oder eine Methylgruppe steht;
- $R_9$ für eine lineare oder verzweigte, zweiwertige Kohlenwasserstoffgruppe mit 1 bis 10 Kohlenstoffatomen und gegebenenfalls einer oder zwei Etherbindungen -O- steht;
- $R_{10}$ für eine lineare oder verzweigte Alkylgruppe mit 1 bis 10 Kohlenstoffatomen steht;
- n für eine ganze Zahl im Bereich von 1 bis 300 steht.

**2.** Dispersion nach dem vorhergehenden Anspruch, wobei das stabilisierend wirkende Blockpolymer drei Blöcke umfasst (Triblock), insbesondere vom Typ löslich/unlöslich/löslich-Triblock.

**3.** Dispersion nach einem der vorhergehenden Ansprüche, wobei der lösliche Block des stabilisierend wirkenden Blockpolymers 50 bis 100 Gew.-% in dem Medium lösliches Monomer umfasst, das alleine oder als Mischung aus den folgenden Monomeren ausgewählt ist:

- Methacrylaten der Formel $CH_2=C(CH_3)-COOR_1$,
worin $R_1$ für eine lineare oder verzweigte $C_8$-$C_{22}$-Alkylgruppe oder eine cyclische Alkylgruppe mit 8 bis 30 Kohlenstoffatomen oder die tert.-Butylgruppe steht;
- Acrylaten der Formel $CH_2=CH-COOR_2$,
worin $R_2$ für eine lineare oder verzweigte $C_8$-$C_{22}$-Alkylgruppe oder eine cyclische Alkylgruppe mit 8 bis 30 Kohlenstoffatomen oder die Isobutylgruppe steht;
- (Meth)acrylamiden der Formel $CH_2=C(CH_3)-CONR_3R_4$ bzw. $CH_2=CH-CONR_3R_4$, worin $R_3$ für ein Wasserstoffatom oder eine lineare oder verzweigte $C_1$-$C_{12}$-Alkylgruppe steht und $R_4$ für eine lineare oder verzweigte $C_8$- bis $C_{12}$-Alkylgruppe steht;
- Di-n-alkylitaconaten der Formel $CH_2=C(CH_2-COO(CH_2)_{n-1}-CH_3)-COO(CH_2)_{n-1}-CH_3$, wobei n für eine ganze Zahl größer gleich 5 steht;
- Vinylestern der Formel $R_5-CO-O-CH=CH_2$, wobei $R_5$ für eine lineare oder verzweigte $C_8$- bis $C_{22}$-Alkylgruppe steht;
- Ethern von Vinylalkohol und einem Alkohol der Formel $R_6O-CH=CH_2$, worin $R_6$ für eine lineare oder verzweigte Alkylgruppe mit 8 bis 22 Kohlenstoffatomen steht;
- ethylenischen Monomeren, deren Estergruppe Silane oder Siloxane enthält, und die nur ein Siliciumatom enthalten, wie (Meth)acryloxypropyltrimethoxysilan;
- Makromonomeren auf Basis von Kohlenstoff mit einer polymerisierbaren Endgruppe.

**4.** Dispersion nach einem der vorhergehenden Ansprüche, wobei der unlösliche Block des stabilisierend wirkenden Blockpolymers 50 bis 100 Gew.-% in dem Medium unlösliches Monomer umfasst, das alleine oder als Mischung aus den folgenden Monomeren sowie deren Salzen ausgewählt ist:

- (Meth)acrylaten der Formel: $CH_2=C(CH_3)-COOR'_1$ bzw. $CH_2=CH-COOR'_1$;
worin $R'_1$ für eine lineare oder verzweigte Alkylgruppe mit 1 bis 6 Kohlenstoffatomen steht, wobei die Gruppe in ihrer Kette ein oder mehrere aus O, N und S ausgewählte Heteroatome enthalten kann und/oder einen oder mehrere Substituenten enthalten kann, die aus -OH, Halogenatomen und
- NR'R" ausgewählt sind, wobei R' und R" gleich oder verschieden sind und aus linearen oder verzweigten $C_1$-$C_4$-Alkylgruppen ausgewählt sind;
- Monomeren mit einer oder mehreren ethylenischen Ungesättigtheiten und mindestens einer Carbonsäurefunktion und deren Salzen;
- ethylenischen Monomeren, deren Estergruppe Silane enthält;

- Polydimethylsiloxanen mit einer Monoacryloyloxy- oder Monomethacryloyloxy-Endgruppe der folgenden Formel:

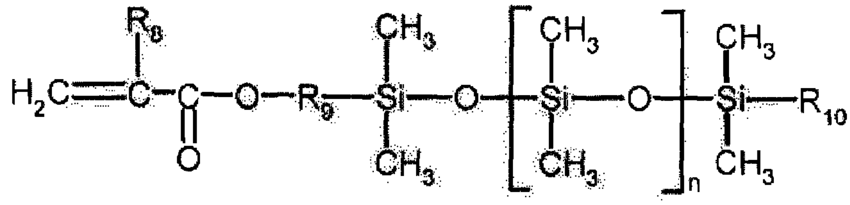

worin:

- $R_8$ für ein Wasserstoffatom oder eine Methylgruppe steht;
- $R_9$ für eine lineare oder verzweigte, zweiwertige Kohlenwasserstoffgruppe mit 1 bis 10 Kohlenstoffatomen und gegebenenfalls einer oder zwei Etherbindungen -O- steht;
- $R_{10}$ für eine lineare oder verzweigte Alkylgruppe mit 1 bis 10 Kohlenstoffatomen steht;
- n für eine ganze Zahl im Bereich von 1 bis 300 steht.

**5.** Dispersion nach einem der vorhergehenden Ansprüche, wobei das flüssige Medium auf Basis von Kohlenstoff mindestens 50 Gew.-%, bezogen auf das Gesamtgewicht des Mediums auf Basis von Kohlenstoff, bei 25°C flüssige Verbindung auf Basis von Kohlenstoff mit einem globalen Löslichkeitsparameter gemäß dem Löslichkeitsraum nach Hansen kleiner gleich 20 $(MPa)^{1/2}$ aufweist, oder eine Mischung derartiger Verbindungen umfasst.

**6.** Dispersion nach einem der vorhergehenden Ansprüche, wobei das flüssige Medium auf Basis von Kohlenstoff mindestens eine Verbindung auf Basis von Kohlenstoff umfasst, die aus den folgenden Verbindungen ausgewählt ist:

- pflanzlichen Ölen aus Estern von Fettsäuren und Polyolen insbesondere Triglyceriden;
- Estern der Formel RCOOR', worin R für den Rest einer höheren Fettsäure mit 7 bis 19 Kohlenstoffatomen steht und R' für eine Kette auf Basis von Kohlenwasserstoff mit 3 bis 20 Kohlenstoffatomen steht;
- flüchtigen oder nichtflüchtigen linearen oder verzweigten $C_8$-$C_{60}$-Alkanen,
- flüchtigen oder nichtflüchtigen nichtaromatischen cyclischen $C_5$-$C_{12}$-Alkanen;
- Ethern mit 7 bis 30 Kohlenstoffatomen;
- Ketonen mit 8 bis 30 Kohlenstoffatomen;
- aliphatischen Fettmonoalkoholen mit 12 bis 30 Kohlenstoffatomen, wobei die Kette auf Basis von Kohlenwasserstoff keine Substitutionsgruppe aufweist,
- Mischungen davon.

**7.** Dispersion nach einem der vorhergehenden Ansprüche, wobei das flüssige Medium auf Basis von Kohlenstoff mindestens Isopropylmyristat, Octyl-dodecanol, $C_5$-$C_{60}$-Isoparaffine, Isododecan, Iso-hexadecan, Isononylisononanoat, Parleam umfasst.

**8.** Dispersion nach einem der vorhergehenden Ansprüche mit einem Trockensubstanzgehalt von 5 bis 40 Gew.-%, insbesondere 6 bis 30 Gew.-% oder sogar 7 bis 25 Gew.-%.

**9.** Kosmetische Zusammensetzung, umfassend mindestens eine Dispersion gemäß einem der Ansprüche 1 bis 8 in einem kosmetisch unbedenklichen Medium.

**10.** Zusammensetzung nach Anspruch 9, wobei die Dispersion in einer solchen Menge vorliegt, dass die Polymer-Trockensubstanzmenge (oder Polymer-Wirkstoffmenge) in der Zusammensetzung 1 bis 70 Gew.-%, vorzugsweise 2 bis 50 Gew.-%, insbesondere 5 bis 40 Gew.-% oder sogar 7 bis 30 Gew.-%, bezogen auf das Gesamtgewicht der Zusammensetzung, ausmacht.

**11.** Zusammensetzung nach einem der Ansprüche 9 bis 10, umfassend mindestens einen Bestandteil, der aus Ölen, Lösungsmitteln, bei Umgebungstemperatur festen Fettsubstanzen, pastösen Fettsubstanzen, Gummen; Wasser; Farbmitteln; Füllstoffen; zusätzlichen Polymeren; Vitaminen, Verdickungsmitteln, Geliermitteln, Spurenelementen, zartmachenden Mitteln, Sequestriermitteln, Duftstoffen, Alkalinisierungsmitteln, Ansäuerungsmitteln, Konservierungsmitteln, Sonnenschutzfiltern, Tensiden, Antioxidantien, Mitteln gegen Haarausfall, Mitteln gegen Schuppen,

Treibmitteln, Ceramiden oder Mischungen davon ausgewählt ist.

**12.** Zusammensetzung nach einem der Ansprüche 9 bis 11, die in Form einer Make-up-Zusammensetzung, insbesondere eines Produkts für den Teint, wie einer Make-up-Grundlage, eines Wangenrouges oder eines Lidschattens; eines Produkts für die Lippen, wie eines Lippenstifts, eines Lippenpflegeprodukts, eines Lipgloss; eines Produkts gegen Falten; eines Rouges, eines Mascaras, eines Eyeliners; eines Augenbrauenschminkprodukts, eines Lippenstifts oder eines Lidschattenstifts; eines Produkts für die Nägel wie eines Nagellacks oder eines Nagelpflegemittels; eines Körperschminkprodukts; eines Haarschminkprodukts (Haarmascaras oder -lacks); einer Zusammensetzung für den Schutz oder die Pflege der Gesichtshaut, des Halses, der Hände oder des Körpers, insbesondere einer Zusammensetzung gegen Falten, gegen Müdigkeit, welches der Haut ein frisches Aussehen verleiht, einer feuchtigkeitsspendenden oder pflegenden Zusammensetzung; eines Sonnenschutzmittels oder eines künstlichen Bräunungsmittels; eines Haar-produkts, insbesondere für die Festigung der Frisur oder zum Formen des Haares, zur Haarpflege, -konditionierung oder -hygiene oder sogar zum Färben des Haares vorliegt.

**13.** Verfahren zur kosmetischen Behandlung, insbesondere für das Schminken, die Reinigung, den Sonnenschutz, das Formen, das Färben oder die Pflege von Keratinsubstanzen, insbesondere der Haut des Körpers oder des Gesichts, der Nägel, des Haares und/oder der Wimpern, bei dem man auf die Substanzen eine kosmetische Zusammensetzung nach einem der Ansprüche 9 bis 12 aufbringt.

**Claims**

**1.** Dispersion of polymer particles in a liquid carbon-based medium, the said particles having a flexible polymeric core and able to be obtained by polymerization:

i) of 60% to 100% by weight, relative to the total weight of monomers, of monomers whose homopolymer has a Tg strictly less than 20°C and which are chosen, alone or as a mixture, from the following monomers, and salts thereof:

- the (meth)acrylates of formula $CH_2=C(CH_3)-COOR$ or $CH_2=CH-COOR$, in which R represents a linear, branched or cyclic, saturated or unsaturated, or even aromatic, alkyl group, comprising 1 to 32 carbon atoms, which may include one or more substituents chosen from -OH, halogen atoms and -NR'R" with R' and R", which may be identical or different, chosen from linear or branched C1-C4 alkyls; and/or interrupted with an oxygen atom;
- ethylenic monomers whose ester group contains silanes, silsesquioxanes, siloxanes or carbosiloxane dendrimers, with the exception of monomers containing only one silicon atom;

ii) and optionally from 0.1% to 40% by weight of monomers whose homopolymer has a Tg of greater than or equal to 20°C;
the glass transition temperature (Tg) of the resulting polymer, forming the flexible core, being strictly less than 20°C;
and the said particles being surface-stabilized with a block stabilizing polymer comprising at least one block that is soluble in the said carbon-based medium and at least one block that is insoluble in the said carbon-based medium, the said insoluble block comprising 50 wt% to 100 wt% of insoluble monomer in the said medium, chosen, alone or as a mixture, from the following monomers, and salts thereof:

- (i) the (meth)acrylates of formula: $CH_2=C(CH_3)-COOR'_1$ or $CH_2=CH-COOR'_1$ in which $R'_1$ represents a group chosen from:

  - a linear or branched alkyl group containing from 1 to 6 carbon atoms, the said group possibly comprising in its chain one or more heteroatoms chosen from O, N and S; and/or possibly comprising one or more substituents chosen from -OH, halogen atoms and -NR'R" with R' and R", which may be identical or different, chosen from linear or branched C1-C4 alkyls; and/or possibly being substituted with at least one polyoxyalkylene group, the said polyoxyalkylene group consisting of a repetition of from 5 to 30 oxyalkylene units; tert-butyl methacrylate and isobutyl acrylate are excluded from this definition;
  - a cyclic alkyl group containing from 3 to 6 carbon atoms, the said group possibly comprising in its chain one or more heteroatoms chosen from O, N and S and/or possibly comprising one or more substituents chosen from OH and halogen atoms;

- (ii) the (meth)acrylamides of formula: $CH_2=C(CH_3)-CONR'_3R'_4$ or $CH_2=CH-CONR'_3R'_4$, in which:

  - $R'_3$ and $R'_4$, which may be identical or different, represent a hydrogen atom or a linear or branched alkyl group containing from 1 to 6 carbon atoms, possibly comprising one or more substituents chosen from -OH, halogen atoms and -NR'R" with R' and R", which may be identical or different, chosen from linear or branched C1-C4 alkyls; or
  - $R'_3$ represents a hydrogen atom and $R'_4$ represents a 1,1-dimethyl-3-oxobutyl group;

- (iii) ethylenically unsaturated monomer(s) comprising at least one carboxylic, phosphoric or sulfonic acid function, and salts thereof;
- (iv) the vinyl esters of formula R'6-COO-CH=$CH_2$ in which $R'_6$ represents a linear or branched alkyl group containing from 1 to 6 atoms or a cyclic alkyl group containing from 3 to 6 carbon atoms and/or an aromatic group;
- (v) ethylenically unsaturated monomers comprising at least one tertiary amine function;
- (vi) the di-n-alkylitaconates of formula $CH_2=C(CH_2-COO(CH_2)_{n-1}-CH_3)-COO(CH_2)_{n-1}-CH_3$, with n being an integer from 0 to 4;
- (vii) ethylenic monomers in which the ester group contains silanes, silsesquioxanes, siloxanes or carbosiloxane dendrimers, with the exception of monomers containing only one silicon atom;
- (viii) PDMS macromonomers, such as polydimethylsiloxanes containing monoacryloyloxy or monomethacryloyloxy end group, and especially those having the following formula:

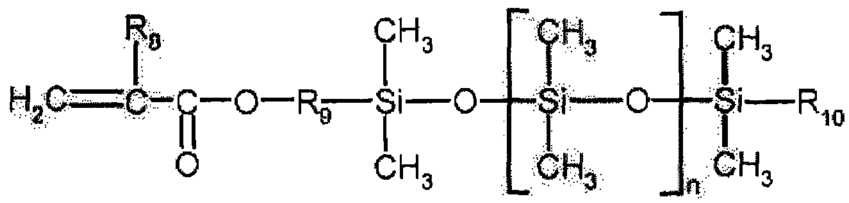

in which:

  - $R_8$ denotes a hydrogen atom or a methyl group;
  - $R_9$ denotes a linear or branched, divalent hydrocarbon-based group containing from 1 to 10 carbon atoms and optionally containing one or two ether bonds -O-;
  - $R_{10}$ denotes a linear or branched alkyl group containing from 1 to 10 carbon atoms;
  - n denotes an integer ranging from 1 to 300.

**2.** Dispersion according to the preceding claim, in which the block stabilizing polymer comprises three blocks (triblock), especially of the soluble/insoluble/soluble triblock type.

**3.** Dispersion according to either of the preceding claims, in which the soluble block of the block stabilizing polymer comprises 50% to 100% by weight of monomer that is soluble in the said medium, chosen, alone or as a mixture, from the following monomers:

- the methacrylates of formula $CH_2=C(CH_3)-COOR_1$ in which $R_1$ represents a linear or branched C8-C22 alkyl group; or alternatively a cyclic alkyl group containing 8 to 30 carbon atoms; or a tert-butyl group;
- the acrylates of formula $CH_2=CH-COOR_2$ in which $R_2$ represents a linear or branched C8-C22 alkyl group; or a cyclic alkyl group containing 8 to 30 carbon atoms; or an isobutyl group;
- the (meth)acrylamides of formula $CH_2=C(CH_3)-CONR_3R_4$ or $CH_2=CH-CONR_3R_4$, in which $R_3$ represents a hydrogen atom or a linear or branched C1-C12 alkyl group and $R_4$ represents a linear or branched $C_8$ to $C_{12}$ alkyl group;
- the di-n-alkylitaconates of formula $CH_2=C(CH_2-COO(CH_2)_{n-1}-CH_3)-COO(CH_2)_{n-1}-CH_3$, with n being an integer greater than or equal to 5;
- the vinyl esters of formula $R_5-CO-O-CH=CH_2$ in which $R_5$ represents a linear or branched $C_8$ to $C_{22}$ alkyl group;
- the ethers of vinyl alcohol and of an alcohol of formula $R_6O-CH=CH_2$ in which $R_6$ represents a linear or branched alkyl group containing from 8 to 22 carbon atoms;
- ethylenic monomers in which the ester group contains silanes or siloxanes, and which contain only one silicon

atom such as (meth)acryloxypropltrimethoxysilane;
- carbon-based macromonomers with a polymerizable end group.

**4.** Dispersion according to one of the preceding claims, in which the insoluble block of the block stabilizing polymer comprises 50% to 100% by weight of monomer that is insoluble in the said medium, chosen, alone or as a mixture, from the following monomers, and salts thereof:

- the (meth)acrylates of formula: $CH_2=C(CH_3)-COOR'_1$ or $CH_2=CH-COOR'_1$;
in which $R'_1$ represents a linear or branched alkyl group comprising from 1 to 6 carbon atoms, the said group possibly comprising in its chain one or more heteroatoms chosen from O, N and S; and/or possibly comprising one or more substituents chosen from -OH, halogen atoms and -NR'R" with R' and R", which may be identical or different, chosen from linear or branched C1-C4 alkyls;
- ethylenically unsaturated monomers comprising at least one carboxylic acid function, and salts thereof;
- ethylenic monomers in which the ester group contains silanes;
- polydimethylsiloxanes containing a monoacryloyloxy or monomethacryloyloxy end group, having the following formula:

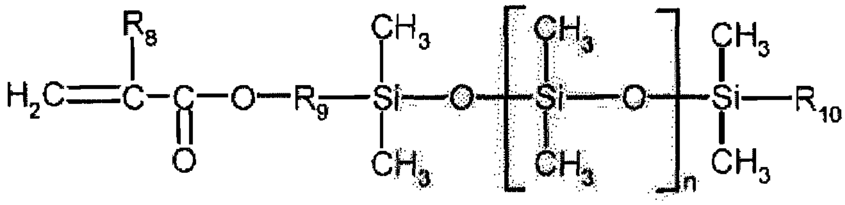

in which:

- $R_8$ denotes a hydrogen atom or a methyl group;
- $R_9$ denotes a linear or branched, divalent hydrocarbon-based group containing from 1 to 10 carbon atoms and optionally containing one or two ether bonds -O-;
- $R_{10}$ denotes a linear or branched alkyl group containing from 1 to 10 carbon atoms;
- n denotes an integer ranging from 1 to 300.

**5.** Dispersion according to one of the preceding claims, in which the liquid carbon-based medium comprises at least 50% by weight, relative to the total weight of the carbon-based medium, of carbon-based compound that is liquid at 25°C, with a global solubility parameter in the Hansen solubility space of less than or equal to 20 $(MPa)^{1/2}$, or a mixture of such compounds.

**6.** Dispersion according to one of the preceding claims, in which the liquid carbon-based medium comprises at least one carbon-based compound chosen from:

- plant oils formed from fatty acid esters of polyols, in particular triglycerides,
- esters of formula RCOOR' in which R represents a higher fatty acid residue comprising 7 to 19 carbon atoms and R' represents a hydrocarbon-based chain comprising from 3 to 20 carbon atoms,
- volatile or non-volatile, linear or branched C8-C60 alkanes;
- volatile or non-volatile, non-aromatic cyclic C5-C12 alkanes;
- ethers containing 7 to 30 carbon atoms;
- ketones containing 8 to 30 carbon atoms;
- aliphatic fatty monoalcohols containing 12 to 30 carbon atoms, the hydrocarbon-based chain not comprising any substitution groups;
- mixtures thereof.

**7.** Dispersion according to one of the preceding claims, in which the liquid carbon-based medium comprises at least isopropyl myristate, octyldodecanol, C5-C60 isoparaffins, isododecane, isohexadecane, isononyl isononanoate, Parleam.

**8.** Dispersion according to one of the preceding claims, which has a solids content of 5% to 40% by weight, especially from 6% to 30% by weight or even from 7% to 25% by weight.

**9.** Cosmetic composition comprising, in a cosmetically acceptable medium, at least one dispersion according to one of Claims 1 to 8.

**10.** Composition according to Claim 9, in which the dispersion is present in an amount such that the amount of polymer solids (or active material) in the composition represents 1% to 70% by weight, preferably 2% to 50% by weight, especially 5% to 40% by weight, or even 7% to 30% by weight, relative to the total weight of the composition.

**11.** Composition according to either of Claims 9 and 10, comprising at least one ingredient chosen from oils, solvents, fatty substances that are solid at room temperature, pasty fatty substances and gums; water; dyestuffs; fillers; additional polymers; vitamins, thickeners, gelling agents, trace elements, softeners, sequestrants, fragrances, acidifying or basifying agents, preserving agents, sunscreens, surfactants, antioxidants, hair-loss counteractants, antidandruff agents, propellants and ceramides, or mixtures thereof.

**12.** Composition according to one of Claims 9 to 11, which is in the form of a makeup composition, especially a complexion product such as a foundation, a face powder or an eyeshadow; a lip product such as a lipstick, a lipcare product or a lip gloss; a concealer product; a blusher, a mascara or an eyeliner; an eyebrow makeup product, a lip pencil or an eye pencil; a nail product such as a nail varnish or a nailcare product; a body makeup product; a hair makeup product (hair mascara or hair lacquer); a composition for protecting or caring for the skin of the face, the neck, the hands or the body, especially an antiwrinkle composition, an anti-fatigue composition for giving the skin radiance, a moisturizing or medicated composition; an antisun or self-tanning composition; a hair product, especially for holding the hairstyle or for shaping the hair; for hair care, conditioning or hygiene; or even for dyeing the hair.

**13.** Cosmetic treatment process, especially for making up, cleansing, sun-protecting, shaping, dyeing or caring for keratin materials, especially bodily or facial skin, the lips, the nails, the hair and/or the eyelashes, comprising the application to the said materials of a cosmetic composition according to one of Claims 9 to 12.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0749747 A
- EP 1428844 A
- EP 0963751 A
- EP 1704854 A
- EP 895467 A
- EP 96459 A
- EP 1347013 A
- US 5625005 A
- EP 749747 A


### Littérature non-brevet citée dans la description


- **BRANDRUP ; IMMERGUT ; GRULKE.** Polymer Handbook. John Wiley, 1999
- **GILLMAN.** *Polymer Letters,* 1967, vol. 5, 477-481
- Solubility parameter values. **DE GRULKE.** Polymer Handbook. 519-559